Europäisches Patentamt

European Patent Office

Office européen des brevets

(19)

(11) Veröffentlichungsnummer: **0 401 508 B1**

(12) **EUROPÄISCHE PATENTSCHRIFT**

(45) Veröffentlichungstag der Patentschrift: **23.11.94**

(51) Int. Cl.5: **C12P 21/02**, C07K 13/00, C12N 15/15

(21) Anmeldenummer: **90108284.2**

(22) Anmeldetag: **01.05.90**

Die Akte enthält technische Angaben, die nach dem Eingang der Anmeldung eingereicht wurden und die nicht in dieser Patentschrift enthalten sind.

(54) **Proteinaseninhibitoren, Verfahren zu ihrer Herstellung sowie diese enthaltende Arzneimittel.**

(30) Priorität: **13.05.89 DE 3915689**
**18.01.90 DE 4001244**

(43) Veröffentlichungstag der Anmeldung:
**12.12.90 Patentblatt 90/50**

(45) Bekanntmachung des Hinweises auf die Patenterteilung:
**23.11.94 Patentblatt 94/47**

(84) Benannte Vertragsstaaten:
**AT BE CH DE DK ES FR GB GR IT LI LU NL SE**

(56) Entgegenhaltungen:
EP-A- 0 255 011

BIOCHEM. JOURNAL, Band 254, 1988; M.W. SWAIM et al., Seiten 171-178&NUM;

BIOL. CHEM. HOPPE SEYLER, Band 366, Mai 1985, Walter de Gruyter & Co.; K. HOCH-STRASSER et al., Seiten 473-478&NUM;

NATURE, Band 313, 10. Januar 1985; M. COURTNEY et al., Seiten 149-151&NUM;

(73) Patentinhaber: **BAYER AG**

**D-51368 Leverkusen (DE)**

(72) Erfinder: **Fritz, Hans, Prof.Dr.**
**Neulinger Strasse 15**
**D-8011 Hohenbrunn (DE)**
Erfinder: **Gebhard, Wolfgang, Dr.**
**Bachstrasse 11**
**D-8063 Unterumbach (DE)**
Erfinder: **Das, Rathindra, Dr.**
**Palkestrasse 15**
**D-5600 Wuppertal 1 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung umfaßt Peptidvarianten auf der Basis der inhibitorisch aktiven Domänen des menschlichen Bikunins (d.i. die inhibitorisch aktive Komponente des Inter-$\alpha$-Trypsininhibitors, bzw. der säurestabile Serum-Trypsininhibitor, bzw. der Harn-Trypsininhibitor), Verfahren, die beschriebenen Peptidvarianten mit Hilfe gentechnologischer Methoden mit Mikroorganismen (Bakterien, niedere Eukaryonten) herzustellen, sowie diese Peptidvarianten enthaltende Arzneimittel. Die Peptidvarianten sind gekennzeichnet durch ihre Fähigkeit Serinproteasen, z.B. pankreatische und granulozytäre Elastase, Kathepsin G oder Plasma-Kallikrein zu hemmen.

Normalerweise werden Proteasen, wenn sie in den Extrazellulärraum gelangen, von potenten endogenen Proteinaseinhibitoren wie dem $\alpha_1$-Proteinaseinhibitor (Travis & Salvesen, Ann. Rev. Biochem. 52, 655, 1983) rasch abgefangen. In bestimmten Situationen kann dieser Schutzmechanismus nicht oder zumindest nicht ausreichend greifen und als Konsequenz kann es zu schweren Krankheitsbildern wie Emphysembildung, septischem Schock, Schocklunge, ARDS, rheumatoider Arthritis, Koagulationsstörungen, Nieren- und Leberversagen u. a. kommen. Spezifisch wirkende Proteinaseinhibitoren sind in diesem Zusammenhang als potentielle Therapeutika von speziellem Interesse. Für eine Anwendung am Menschen sind dabei Proteinaseinhibitoren von besonderem Interesse, deren Aminosäuresequenzen natürlichen humanen Inhibitoren ähnlich sind. Dies gilt insbesondere für Langzeittherapien wie die Behandlung von $\alpha_1$-Proteinaseinhibitor-Defizienzen (Emphysembildung) zur Vermeidung toxischer oder allergischer Nebenwirkungen.

Obwohl der $\alpha_1$-Proteinaseinhibitor der natürliche Gegenspieler der neutrophilen Elastase ist, die es bei einem entzündlichen Geschehen extrazellulär primär zu hemmen gilt, ist er für eine therapeutische Anwendung aus mehreren Gründen nicht optimal geeignet. Sein relativ hohes Molekulargewicht von 53 000 d zwänge zum Einsatz unphysiologisch hoher Gewichtsmengen des Inhibitors. Zwar wären die erforderlichen Mengen auf gentechnischem Weg zugänglich, doch erforderte die verminderte biologische Halbwertzeit eines nicht in physiologischer Weise glykosylierten rekombinanten Proteins in der Zirkulation (Matheson et al., J. Biol. Chem. 261, 10404, 1986) den Einsatz noch größerer Mengen des Inhibitors. Ohnehin gilt der Inhibitor als proteolysierbar und oxidierbar.

Beide Eigenschaften verringern die Konzentration der aktiven Spezies weitere. Lediglich die Oxidierbarkeit wäre auf gentechnischem Weg durch Substitution des Methioninrestes im reaktiven Zentrum des Inhibitors ($P_1$-Position) durch z.B. Leucin (McCourtney et al., Nature 313,149, 1985) zu beseitigen.

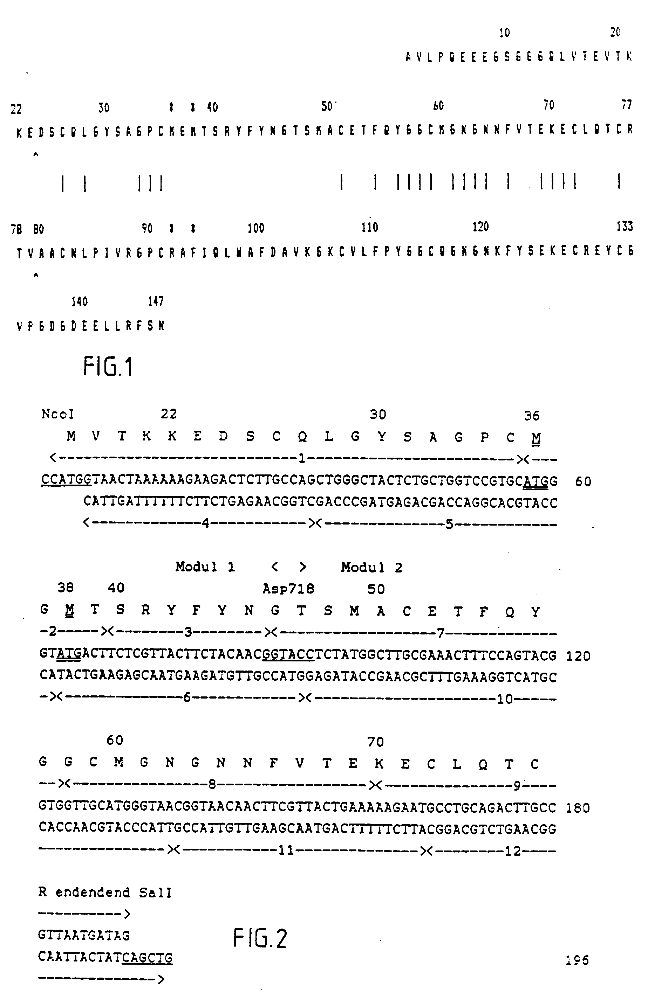

Ziel der vorliegenden Erfindung ist es, Proteinaseinhibitoren z.B. gegen humane Leukozytenelastase, Kathepsin G oder Plasma-Kallikrein mit deutlich kleinerer Molekülmasse für die Therapie menschlicher Erkrankungen zu entwickeln. Es galt dabei zu überprüfen, ob durch Modifikation bereits bekannter menschlicher Inhibitoren Proteinaseinhibitoren mit einem erwünschten Hemmspektrum maßgeschneidert werden können. Demnach wären die inhibitorisch aktive Untereinheit des menschlichen Inter-$\alpha$-Trypsininhibitors ITI (Schreitmüller et al., Biol. Chem. Hoppe-Seyler, 368, 963, 1987; Gebhard et al., Biol. Chem. Hoppe-Seyler 369, 19, 1988; Gebhard et al., FEBS Lett. 229, 63, 1988; Gebhard et al., Euro. J. Biochem. im Druck), die im folgenden als Bikunin bezeichnet wird (Abb. 1), sowie die strukturidentischen Kunitztyp-Trypsininhibitoren des Serums (STI) und des Harns (UTI) als geeignete Grundmoleküle denkbar, wenn es gelänge, durch Austausch einiger weniger Aminosäurereste eine gezielte Änderung des natürlichen Hemmspektrums dieser Inhibitoren zu erreichen (Abb.1). Sie sind das Ergebnis der proteolytischen Reifung des Primärtranslationsprodukts eines einzigen Gens (Kaumeyer et al., Nucleic Acids Res. 14, 7839, 1986) und bestehen aus einem tryptisch abspaltbaren N-terminalen Peptid (Aminosäurereste 1-21) und zwei aufeinanderfolgenden, strukturverwandten Domänen (Domäne 1 ist die N-terminale Domäne, definitionsgemäß Aminosäurereste 22-77, und Domäne 2 der C-terminale Teil des Proteins, definitionsgemäß Aminosäurereste 78-147), die nach Behandlung mit Trypsin auch einzeln zu erhalten sind. Beide Domänen besitzen Proteinaseinhibitor-Aktivität unterschiedlicher Hemmspezifität (Gebhard & Hochstraßer in: Proteinase Inhibitors, Barrett & Salvesen, eds., Elsevier, 1986, p 375).

In Abhängigkeit von der jeweiligen physiologischen Situation kann das Akutphasenprotein Bikunin sowohl in komplexierter Form (im ITI oder im Komplex mit Immunglobulinen) als auch in nicht-komplexierter Form vorliegen, die dann als STI nachweisbar ist. UTI ist renal filtrierter STI. Die einzelnen Inhibitoren unterscheiden sich demnach nur dadurch, daß sie entweder mit anderen Proteinen assoziiert sind oder nicht, bzw. in verschiedenen Körperflüssigkeiten nachgewiesen werden.

Die spezifischen und potenten Inhibitoren z.B. der neutrophilen Elastase, des Kathepsin G oder Plasma-Kallikreins auf der Basis der Bikunin-Domänen können erhalten werden durch den Ersatz von Aminosäuren in Position $P_1$ des reaktiven Zentrums der Domäne 1 oder der Domäne II des Inhibitors.

Des weiteren wurde gefunden, daß zusätzliche Austausche in den Domänen, so z.B. insbesondere in der Position $P_2'$, die Hemmeigenschaften zusätzlich verbessern können.

Demgemäß bezieht sich die vorliegende Erfindung allgemein auf Varianten von Kunitztypinhibitoren, deren Hemmspektren aufgrund des Austauschs eines natürlichen Aminosäurerestes nicht nur in Position $P_1$, sondern auch in $P_2'$-Position des reaktiven Zentrums durch einen anderen natürlichen Aminosäurerest verändert wurden, speziell auf solche Inhibitoren, die auf der Basis der einzelnen oder miteinander verknüpften Domänen des menschlichen Bikunins erhalten wurden, soweit durch Ersatz der natürlichen Aminosäurereste in den Positionen $P_1$ und/oder $P_2'$ der reaktiven Zentren einzelner oder beider Domänen durch irgendeine der natürlichen Aminosäuren, insbesondere aber durch Aminosäuren aus der Gruppe Ala, Gly, Ile, Leu, Arg, Phe, Val, Tyr, Trp, Lys die Hemmeigenschaften des natürlichen Inhibitors bzw. seiner Einzeldomänen verändert und/oder verbessert wurden. Ferner bezieht sich die Erfindung auch auf die den Inhibitorvarianten zugrundeliegenden Genkonstruktionen, unabhängig von der jeweils getroffenen Kodonauswahl und der Verknüpfung von Bereichen natürlicher cDNA mit solchen synthetischer DNA.

Des weiteren bezieht sich die vorliegende Erfindung auch auf Bikunin-Varianten, in denen neben Austauschen in einer oder in beiden $P_1$-Positionen sowie in einer oder in beiden $P_2'$-Positionen noch weitere Austausche in anderen Positionen vorliegen. Solche zusätzlichen Austausche können die erwünschten Hemmeigenschaften weiter verbessern, zu günstigerem pharmakokinetischen Verhalten führen, die Halbwertszeit in vivo verlängern oder zu einer günstigeren technischen Herstellbarkeit führen. Schließlich umfaßt die vorliegende Erfindung auch Bikunin-Varianten mit nach Art und Ausmaß unterschiedlichen N-terminalen und C-terminalen Peptidanteilen außerhalb der für die Ausbildung der Grundstrukturen notwendigen Cysteinreste in den Positionen 26 und 76 (Inhibitordomäne 1) und 82 und 132 (Inhibitordomäne 2). Die ursprünglich definierten Domänengrenzen ($Lys^{22}$ und $Arg^{77}$ der Inhibitordomäne 1 und $Thr^{78}$ und $Asn^{147}$ der Inhibitordomäne 2) sind aufgrund der durch Trypsin erreichbaren Auftrennung in funktionelle Peptide definiert worden und stimmen nicht exakt mit den inzwischen bestimmten Exonbereichen überein (Vetr et al., FEBS Lett. im Druck, 1989). Demnach wären die beiden Inhibitordomänen von den Aminosäureresten $Asp^{24}$ und $Val^{79}$ bzw. $Ala^{80}$ und $Asp^{137}$ begrenzt (Abb.1). Abhängig vom verwendeten Expressionssystem können einzelne Genkonstruktionen für N- bzw. C-terminale Peptidverlängerungen kodieren, unabhängig davon, ob diese ganz oder teilweise in der endgültigen Inhibitorvariante erhalten bleiben oder lediglich vorübergehend als Teil eines Vorläuferproteins fungieren und ob diese Verlängerungen dem natürlichen Bikunin oder einem Fremdprotein zuzuordnen sind.

Beispielsweise kann die N-terminale Sequenz $Met^{18}$-$Thr^{19}$-$Val^{20}$-$Lys^{21}$, die mit Ausnahme des Methioninrests mit der natürlichen Aminosäuresequenz des Bikunins identisch ist, vorteilhafterweise dazu verwendet werden, durch Spaltung mit Trypsin einheitliches Produkt zu erhalten, das der Zusammensetzung der ursprünglich definierten Domäne 1 entspricht. Auch aus Konstruktionen mit N-terminalem Fremdproteinanteil können auf diesem Weg Bikunindomänen glatt abgetrennt werden. Bei direkter Expression (ohne N-terminalem Fremdproteinanteil) ist auch eine partielle, bzw. unter bestimmten Bedingungen auch vollständige Abspaltung des N-terminalen Methionins zu erwarten. Zur Vermeidung einer möglichen Entfernung des C-terminalen Arginins der einzelköpfigen Konstruktionen der Domäne 1 bei therapeutischen Anwendungen sind unter Umständen C-terminale Extensionen bevorzugt.

Die Expression von Varianten des Bikunins oder von Fragmenten von Bikuninvarianten kann erfolgreich mit bakteriellen oder eukaryontischen Systemen durchgeführt werden. So bietet sich unter den bakteriellen Systemen z.B. die Expression in Escherichia coli K 12 Stämmen an; entweder in nicht-fusionierter Form im Cytoplasma, als intrazellulär gebildetes Fusionsprotein verbunden mit einem geeigneten Fusionspartner, z.B. dem N-terminalen Teil der MS2-Replikase, oder auch als hemmaktives und in den Periplasma-Raum sekretiertes Produkt unter Verwendung geeigneter Signalpeptide z.B. der OmpA-Signalsequenz.

Unter den eukaryontischen Systemen eignen sich z.B. Hefesekretionssysteme, bei denen das Expressionsprodukt durch eine geeignete Leadersequenz z.B. der alpha-Faktor-Präpro-Sequenz durch den Sekretionsweg geschleust und als hemmaktive Substanz in das Kulturmedium abgegeben wird. Desweiteren können auch Hefeexpressionssysteme verwendet werden, die zu einer intrazellulären Synthese führen.

Darüber hinaus können aber auch zahlreiche andere pro- und eukaryontische Expressionssysteme verwendet werden etwa unter Verwendung von Bacillus-, Staphyllococcus-, Hansenula-, Aspergillus- oder anderen Wirtsstämmen.

Soll eine Glykosylierung ähnlich der in Säugetieren vorhandenen erzielt werden, sind richtig glykosylierende Systeme zu wählen. Nicht-glykosylierte Bikuninvarianten werden in prokaryontischen Expressionssystemen erhalten. Hefeexpressionssysteme führen in der Regel zu einer vom Säugetierglykosylierungsmuster abweichenden Glykosylierung. Nichtglykosylierte mit Hefeexpressionssystemen gewonnene Bikuninvarianten lassen sich durch Verwendung deglykosylierender Enzyme oder durch Expression nicht-glykosylierbarer Varianten herstellen.

Die vorliegende Erfindung umfaßt pharmazeutische Zubereitungen, die außer nicht-toxischen, inerten, pharmazeutisch geeigneten Exzipienten eine oder mehrere Verbindungen gemäß der Erfindung umfassen oder aus einer oder mehreren aktiven Verbindungen gemäß der Erfindung bestehen, sowie Verfahren zur Herstellung dieser Zubereitungen.

Die vorliegende Erfindung umfaßt auch pharmazeutische Zubereitungen in Dosiseinheiten. Dies bedeutet, daß die Zubereitungen in Form individueller Teile vorliegen, z.B. als Tabletten, beschichtete Tabletten, Kapseln, Pillen, Suppositorien und Ampullen, deren Gehalt an Wirkstoff einer Fraktion oder einem Vielfachen einer individuellen Dosis entspricht. Die Dosiseinheiten können z.B. enthalten: ein, zwei, drei oder vier individuelle Dosen oder eine Hälfte, ein Drittel oder ein Viertel einer individuellen Dosis. Eine individuelle Dosis enthält vorzugsweise die Menge an Wirkstoff, die bei einer Verabreichung gegeben wird, und die gewöhnlich dem Ganzen, der Hälfte oder einem Drittel oder einem Viertel der Tagesdosis entspricht.

Unter nicht-toxischen, inerten, pharmazeutisch geeigneten Exzipienten sind zu verstehen: feste, halbfeste oder flüssige Verdünner, Füller und Formulierungshilfsmittel aller Art.

Tabletten, beschichtete Tabletten, Kapseln, Pillen, Granulate, Suppositorien, Lösungen, Suspensionen und Emulsionen, Pasten, Salben, Gele, Cremes, Lotionen, Pulver und Sprays können als bevorzugte pharmazeutische Zubereitungen genannt werden.

Tabletten, beschichtete Tabletten, Kapseln, Pillen und Granulate können den Wirkstoff oder die Wirkstoffe zusammen mit üblichen Exzipienten, wie (a) Füller und Streckmittel, z.B. Stärken, Lactose, Sucrose, Glucose, Mannit und Siliziumoxid, (b) Bindemittel, z.B. Carboxymethylcellulose, Alginate, Gelatine und Polyvinylpyrrolidon, (c) anfeuchtende Mittel, z.B. Glycerin, (d) disintegrierende Mittel, z.B. Agar-Agar, Kalziumcarbonat und Natriumcarbonat, (e) Lösungsverzögerer, z.B. Paraffin und (f) Absorptionsbeschleuniger, z.B. quaternäre Ammoniumverbindungen, (g) Benetzungsmittel, z.B. Cetylalkohol und Glycerinmonostearat, (h) Absorbentien, z.B. Kaolin und Bentonit und (i) Schmiermittel, z.B. Talk, Kalzium- und Magnesiumstearat und feste Polyethylenglykole, oder Gemische der unter (a) bis (i) aufgeführten Substanzen, enthalten.

Die Tabletten, beschichteten Tabletten, Kapseln, Pillen und Granulate können mit üblichen Überzügen und Umhüllungen versehen werden, die gegebenenfalls Opazifizierungsmittel enthalten; sie können eine Zusammensetzung aufweisen, so daß die Freisetzung des Wirkstoffes oder der Wirkstoffe nur, oder vorzugsweise, in einem bestimmten Teil des Intestinaltraktes, gegebenenfalls in verzögerter Form, erfolgt, wobei Beispiele für geeignete Zubereitungen zum Einbetten polymere Substanzen und Wachse bilden.

Die aktive Verbindung oder Verbindungen, gegebenenfalls zusammen mit einem oder mehreren der vorstehend genannten Exzipienten, kann bzw. können auch in mikroverkapselter Form vorliegen.

Suppositorien können außer dem Wirkstoff bzw. den Wirkstoffen enthalten: übliche wasserlösliche oder wasserunlösliche Exzipienten, z.B. Polyethylenglykole, Fette, z.B. Kakaofett und höhere Ester (z.B. $C_{14}$-Alkohol mit $C_{16}$-Fettsäure) oder Gemische dieser Substanzen.

Salben, Pasten, Cremes und Gele können zusätzlich zu dem Wirkstoff oder den Wirkstoffen übliche Exzipienten enthalten, z.B. tierische und pflanzliche Fette, Wachse, Paraffine, Stärke, Tragacanth, Cellulosederivate, Polyethylenglykole, Silikone, Bentonite, Siliziumdioxid, Talk und Zinkoxid, oder Gemische dieser Substanzen.

Pulver und Sprays können außer dem Wirkstoff oder den Wirkstoffen übliche Exzipienten enthalten, z.B: Lactose, Talk, Siliziumdioxid, Aluminiumhydroxid, Kalziumsilikat und Polyamidpulver, oder Gemische dieser Substanzen. Sprays können außerdem die üblichen Treibmittel enthalten, z.B. Chlorfluorkohlenwasserstoffe.

Lösungen und Emulsionen können außer dem Wirkstoff oder den Wirkstoffen übliche Exzipienten umfassen, wie Lösungsmittel, solubilisierende Agentien und Emulgiermittel, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsamenöl, Erdnußöl, Maiskeimöl, Olivenöl, Rizinusöl und Sesamöl, Glycerin, Glycerinformal, Tetrahydrofurfurylalkohol, Polyethylenglykole und Fettsäureester von Sorbitan, oder Gemische dieser Substanzen.

Zur parenteralen Verabreichung können die Lösungen und Emulsionen auch in einer sterilen Form vorliegen, die mit Blut isoton ist.

Suspensionen können zusätzlich zum Wirkstoff oder den Wirkstoffen übliche Exzipienten enthalten, wie flüssige Verdünner, z.B. Wasser, Ethylalkohol oder Propylenglykol, Suspendierungsmittel, z.B. ethoxylierte Isostearylalkohole, Polyoxyethylensorbit und Sorbitanester, mikrokristalline Cellulose, Aluminium-metahydroxid, Bentonic, Agar-Agar und Tragacanth, oder Gemische dieser Substanzen.

Die genannten Formulierungsformen können auch enthalten: Farbstoffe, Konservierungsmittel und Additive, die den Geruch oder Geschmack verbessern, z.B. Pfefferminzöl und Eukalyptusöl, und Süßungsmittel, z.B. Saccharin.

Die therapeutisch wirksamen Verbindungen sollten in den vorstehend genannten pharmazeutischen Zubereitungen vorzugsweise in einer Konzentration von 0,1 bis 99,5 Gew.-%, vorzugsweise von ca. 0,5 bis 95 Gew.-%, bezogen auf das Gesamtgemisch, vorliegen.

Die vorstehend genannten pharmazeutischen Zubereitungen können zusätzlich zu den Verbindungen gemäß der Erfindung auch andere pharmazeutische Wirkstoffe enthalten.

Die vorstehend genannten pharmazeutischen Zubereitungen werden in üblicher Weise nach bekannten Verfahren hergestellt, z.B. durch Vermischen des Wirkstoffes oder der Wirkstoffe mit dem Exzipienten oder den Exzipienten.

Die Wirkstoffe oder die pharmazeutischen Zubereitungen können lokal, oral, parenteral, intraperitoneal und/oder rektal verabreicht werden, vorzugsweise erfolgt dies oral oder parenteral, wie z.B. intravenös oder intramuskulär.

Im allgemeinen hat es sich sowohl in der Humanmedizin als auch in der Tiermedizin als vorteilhaft erwiesen, den Wirkstoff oder die Wirkstoffe gemäß der Erfindung in den Gesamtmengen von ca. 0,5 bis ca. 500, vorzugsweise von 4 bis 100 mg/kg Körpergewicht alle 24 Stunden, gegebenenfalls in Form von mehreren individuellen Verabreichungen, zu geben, um die besten Ergebnisse zu erzielen. Eine individuelle Verabreichung enthält den Wirkstoff oder die Wirkstoffe gemäß der Erfindung vorzugsweise in Mengen von ca. 1 bis ca. 250, insbesondere 3 bis 60 mg/kg Körpergewicht. Es kann jedoch erforderlich sein, von der genannten Dosis abzuweichen, insbesondere in Abhängigkeit von der Natur und dem Körpergewicht des zu behandelnden Individuums, der Natur und Schwere der Krankheit, der Art der Zubereitung und der Verabreichung der Medizin, und der Zeit oder dem Intervall, über den die Verabreichung stattfindet.

So kann es in einigen Fällen genügen, mit weniger als der vorstehend genannten Menge an Wirkstoff auszukommen, während in anderen Fällen die vorstehend genannte Menge an Wirkstoff überschritten werden muß. Die speziell erforderliche optimale Dosis und die Verabreichungsart der Wirkstoffe kann in einfacher Weise vom Fachmann auf der Basis seines Fachwissens entschieden werden.

Methoden

Zur Spaltung von DNA mit Restriktionsenzymen, zum Auffüllen von 5′-überstehenden Enden mit dNTP in Gegenwart von DNA-Polymerase I (Klenow-Fragment), zum Verdau mit Mungbohnen-Nuklease, Gelelektrophorese von DNA, Isolierung von DNA-Fragmenten, Ligieren von DNA-Fragmenten, zur Transformation von E.coli und zur Koloniehybridisierung wurden unter Berücksichtigung der Herstellerangaben (Restriktionsenzyme der Firmen Boehringer Mannheim (Mannheim), Biolabs (Schwalbach), Pharmacia (Freiburg); Mungbohnen-Nuklease, Pharmacia, und kompetente E.coli-Zellen, BRL, Eggenstein) Standardmethoden verwendet wie sie bei Maniatis et al, Molecular Cloning, Cold Spring Harbor (1982) beschrieben sind.

Chemische Synthese der Oligonukleotide

Die Oligonukleotide wurden am Pharmacia Gene Assembler™ mit Hilfe etablierter Phosphoramidit-Chemie ($\beta$-Cyanoethyl-N,N-diisopropylphosphoramidit) synthetisiert und durch denaturierende Polyacrylamid-Gelelektrophorese gereinigt.

DNA-Sequenzierung

Zur Verifizierung der DNA-Sequenz der einzelnen Genkonstruktionen wurde doppelsträngige DNA jeweils in beiden Strängen nach der Methode von Chen & Seeburg (DNA, 4, 165, 1985) direkt sequenziert.

Hefe-Transformation

Es wurden 100 ml einer Hefezellsuspension des Stamms SC106 (MAT-alpha, hom3, gal2, his6, ura3; Stamm S2207A, Yeast Genetics Stock Center, University of California, Berkeley, CA 94720, USA) mit einer Zellkonzentration von $2 \times 10^7$ pro ml abzentrifugiert; das Zellsediment wurde einmal mit 5 ml TE-Puffer (10 mM Tris x HCl, pH 7,5, 1 mM EDTA) und dann mit 5 ml LiA-Puffer (0,1 M Lithium-Acetat in TE-Puffer) gewaschen. Die Zellen wurden dann in 1 ml LiA-Puffer suspendiert und 1 Stunde bei 30°C inkubiert. Die so erhaltenen kompetenten Zellen konnten für 1 Tag bei 4°C gelagert werden. Die Transformation wurde folgendermaßen durchgeführt:

Zu 0,1 ml Zellsuspension wurden 10 $\mu$l der Plasmidlösung (1 - 5 $\mu$g DNA) und 15 $\mu$l einer Carrier-DNA (denaturierte DNA aus Heringssperma, 3 mg/ml) gegeben. Nach einer Inkubation für 30 min bei 30°C wurden 0,7 ml Polypropylenglykol (40 % Polypropylenglykol 3350 in LiA-Puffer) zugegeben und danach weitere 60 min bei 30°C inkubiert. Die Zellen wurden danach einem Hitzeschock unterworfen (5 min, 42°C) und anschließend 4 sec in einer Eppendorf Mikrofuge abzentrifugiert. Das Zellpellet wurde zweimal mit jeweils 0,5 ml TE-Puffer gewaschen, die Zellen wurden dann in 0,1 ml TE-Puffer suspendiert und auf Selektiv-Nährboden ausplattiert. Transformanten wurden nach 3 Tagen erhaltene.

## Wachstum von Transformanten und Analyse von Sekretionsprodukten

Transformanten wurden in SD-Medium (0,67 % Yeast Nitrogen Base ohne Aminosäuren, 2 % D-Glucose) supplementiert mit Threonin, Methionin und Histidin (jeweils 20 mg/Ltr.) bei 30°C kultiviert. Nach Erreichen einer ausreichenden Zelldichte wurden die Zellen abzentrifugiert und die Trypsin- oder Elastase-hemmende Aktivität im Kulturüberstand gemessen.

## Polyacrylamid-Gelelektrophorese

Proteine wurden üblicherweise mit SDS-Polyacrylamid-Gelelektrophorese (Laemmli, Nature 277, 680, 1970) und Färbung mit Coomassie Brilliant Blau nachgewiesen.

## Aminosäure-Analyse

Etwa 1 nMol Protein wurde in Gegenwart von 200 $\mu$l 6M HCl, 0,05 % $\beta$-Mercaptoethanol unter Vakuum bei 110°C 22 h inkubiert. Die Hydrolysate wurden getrocknet, in 150 $\mu$l 0,2 M Natrium-Citratpuffer pH 2,2 gelöst und filtriert. Die Aminosäureanalyse wurde an einem Biotronic LC 5000 Aminosäureanalysator mit Fluoreszenz-Detektor und Shimadzu C-R2AX Integrator durchgeführt. Die Aminosäure wurden nach Reaktion mit o-Phtalaldehyd entsprechend der Literatur quantifiziert (Benson & Hare, Proc. Natl. Acad. Sci, USA 72, 619 (1975)).

## Aminosäure-Sequenzierung

1-2 nMol Protein wurden nach Lösen in 30 $\mu$l Trifluoressigsäure auf Polybren-behandelte Glasfaserfilter aufgebracht und im Gasphasensequenator (Applied Biosystems) nach Hewick et al., J. Biol. Chem. 256 7990 (1981) sequenziert. Phenylthiohydantoin-Derivate wurden mit Hilfe einer cyano-HPLC-Säule (DuPont) wie bei Beyreuther et al., Modern Methods in Protein Chemistry, 303-325, Walter de Gruyter, Berlin (1983) beschrieben mit Hilfe eines Waters HPLC-Sytems separiert und analysiert.

## Trypsin-Hemmtest

Die Trypsinaktivität wurde nach Geiger & Fritz, Methods of Enzymatic Analysis, Vol. V, 3rd ed., Bergmeyer (ed), Verlag Chemie, Weinheim (1984), p.121 mit Benzoyl-L-arginin-p-nitroanilid als Substrat bestimmt. Das freigesetzte p-Nitroanilin wurde spektrophotometrisch bei 405 nm gemessen. Enzym und Inhibitor wurden vor der Zugabe des Substrats 15 min vorinkubiert.

## Elastase-Hemmtest

Humane Leukozyten-Elastase kam von Elastin Products Company, Inc, P.O. Box 147, Pacific, Miss 63069/USA. Als Substrat wurde MeOSuc-Ala-Ala-Pro-Val-pNA (Bachem, Bubendorf, Schweiz) verwendet. Die Testbedingungen sind in Tabelle 1 angegeben. Allgemein wurden die Inhibitorproben nach Verdünnen mit Testpuffer und Zugabe von Enzym präinkubiert. Die Reaktion wurde durch Zugabe von Substrat (gelöst in DMSO in einer Konzentration von 0,1 M und mit Puffer auf die Konzentration der Stocklösung gebracht) gestartet und die Freisetzung von p-Nitroanilin aus dem Substrat bei 405 nm kontinuierlich verfolgt. 100 %-Werte wurden in entsprechenden Tests ohne Inhibitoren bestimmt. Die Hemmung (in Prozent) wurde aus folgender Gleichung errechnet.

$$\% \text{ Inhibierung} = 100 \times \left(1 - \frac{\Delta \text{ OD in Gegenwart von Inhibitor}}{\Delta \text{ OD in Abwesenheit von Inhibitor}}\right)$$

Tabelle 1

| Bedingungen des Elastase-Hemmtests (Nakijima et al., J. Biol. Chem. 254, 4027, 1979) | |
| --- | --- |
| Puffer | 0,2M Tris/HCl, pH 8,0 + 0,1 % Tween 80 |
| Gesamtvolumen nach Substratzugabe | 0,65 ml |
| Enzymmenge/Test | 50 ng |
| Präinkubationszeit bei Raumtemperatur | 30 min |
| Substrat | MeO-Suc-Ala-Ala-Pro-Val-pNA |
| Stocklösung | 0,065 M |
| Menge/Test | 0,1 ml |
| Testtemperatur | 30 °C |

Beispiel 1

Konstruktion der Bikunin-Genvarianten und Expression in E. coli

Die einzelnen Bikunin-Genvarianten basieren zum einen auf der natürlichen mRNA zum anderen auf synthetischen Genen der einzelnen Domänen oder Teilen davon. Die cDNA-Klone wurden aus geeigneten cDNA-Banken durch Hybridisierung mit Oligonukleotidsonden (Kaumeyer et al., Nucleic Acids Res. 14, 7839-7849, 1986) oder durch Sichten mit Antikörpern gegen humanen Inter-$\alpha$-Trypsininhibitor (Schreitmüller et al., Biol. Chem. Hoppe-Seyler 368,963-970, 1987) erhalten. Die synthetischen Gene (1. Domäne) bzw. Genabschnitte (2. Domäne) wurden wie folgt erhalten:

Konstruktion des des[$^{1-17,78-147}$]-Met$^{18}$-Xaa$^{36}$-Xaa$^{38}$-Bikunin-Gens, mit z.B. Xaa = Ile, Leu, Met, Val

Die Gene für die 1. Domäne (des des[$^{1-17,78-147}$]-Met$^{18}$-Bikunin) und ihre Varianten (des des-[$^{1-17,78-147}$]-Met$^{18}$-Xaa$^{36}$-Xaa$^{38}$-Bikunin, mit z.B. Xaa = Ile, Leu, Met, Val) wurden aus zwei vollsynthetisch erzeugten Modulen zusammengesetzt, die durch Spaltung mit der geeigneten Kombination der Restriktionsnukleasen NcoI, Asp718 und SaI einzeln oder zusammen aus diesen Genen entfernt werden können. Die Konstruktion erleichtert die Handhabung der Gene in verschiedenen Vektorsystemen und über die interne Asp718-Restriktionsspaltstelle den Austausch der Module zur Herstellung der Varianten (Abb.2).

Modul 1 umfaßt die Oligonukleotide 1-6, Modul 2 die Oligonukleotide 7-12. Die Konstruktion von Xaa$^{36}$-Xaa$^{38}$-Varianten bedarf lediglich des Austauschs von Modul 1. Zur Konstruktion der Modul-1-Varianten wurden, soweit nötig, die Oligonukleotide 2,3,5 und 6 durch Oligonukleotidvarianten ersetzt, in denen das Kodon für Methionin (ATG) durch solches für Isoleucin (ATC), Leucin (CTG oder Valin (GTT) bzw. im Gegenstrang deren komplementäre Sequenz ersetzt worden war. Die N-terminale Sequenz V-T-K ist Teil des natürlichen N-terminalen Peptids. Einzige Fremdaminosäure ist das Start-Methionin, deren Abspaltung bei direkter Expression des Gens in E. coli in vivo erreicht werden kann.

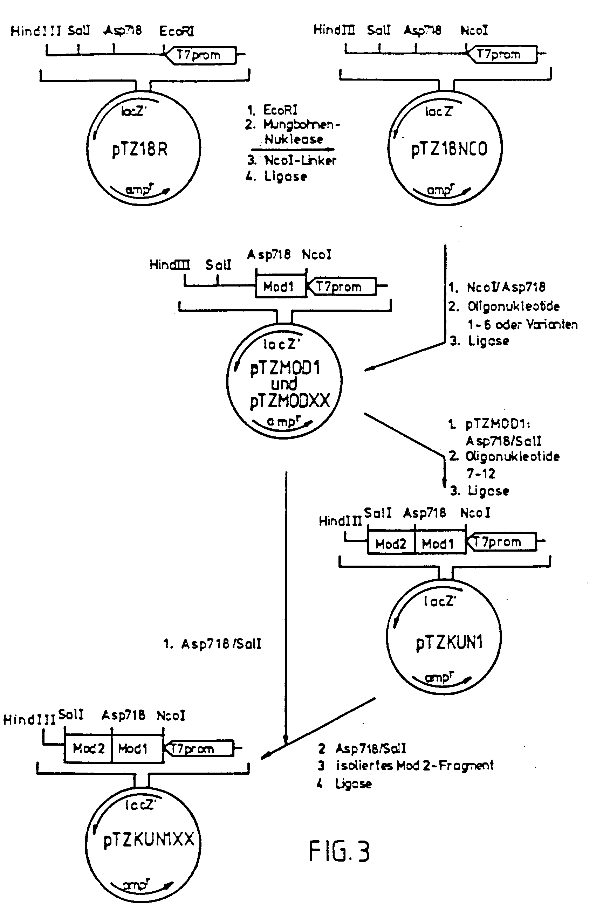

Die Klonierungsstrategie ist in Abb.3 skizziert. Plasmid pTZ18NCO wurde durch Spaltung von pTZ18R (Pharmacia) mit EcoRI, Behandlung mit Mungbohnen-Nuklease, Ligieren mit der palindromischen Sequenz 5'-ACCATGGT-3' und Transformation in E. coli DH5 hergestellt. Die Konstruktion wurde durch Sequenzanalyse verifiziert.

Die Plasmide pTZMODI und pTZMOD1XX (XX = IL, LM, MM, LL, VL) wurden nach Spaltung von Plasmid pTZ18NCO mit NcoI und Asp718, Ligieren des linearisierten Plasmids mit den mit Polynukleotidkinase phosphorylierten Oligonukleotiden 1-6 (Modul 1, Abb.2) bzw. den entsprechenden Oligonukleotidvarianten, Transformation in E. coli DH5, Koloniehybridisierung mit radioaktiv markiertem Oligonukleotid 4 und Isolierung der Plasmid-DNA durch Sequenzieren charakterisiert.

Plasmid pTZKUN1 wurde nach Spaltung von Plasmid pTZMOD1 mit Asp718 und SalI, Ligieren des linearisierten Plasmids mit den mit Polynukleotidkinase phosphorylierten Oligonukleotiden 7-12 (Modul 2, Abb.2), Transformation in E. coli DH5 und Koloniehybridisierung mit radioaktiv markiertem Oligonukleotid 11 isoliert und durch Sequenzieren der Plasmid-DNA charakterisiert.

Zur Konstruktion der Plasmide pTZKUN1XX (XX = IL, LM, MM, LL, VL) wurde anstelle der einzelnen Oligonukleotide 7-12 des Moduls 2 das aus pTZKUN1 mit Asp718 und SalI abgetrennte Fragment dieses Moduls verwendet und ansonsten analog zu den bereits beschriebenen Konstruktionen verfahren.

Konstruktion von des des$[^{1-17}]$-Met$^{18}$-Xaa$^{36}$-Xaa$^{38}$-Bikunin-Genen

Bikuningene des Typs des des$[^{1-17}]$-Met$^{18}$ Xaa$^{36}$-Xaa$^{38}$-Bikunin sind doppelköpfige Varianten mit Aminosäureaustauschen nur in der 1. Domäne. Sie wurden durch Kondensation des synthetischen Gens der 1. Domäne bzw. seiner Varianten an ein natürliches Genfragment der 2.

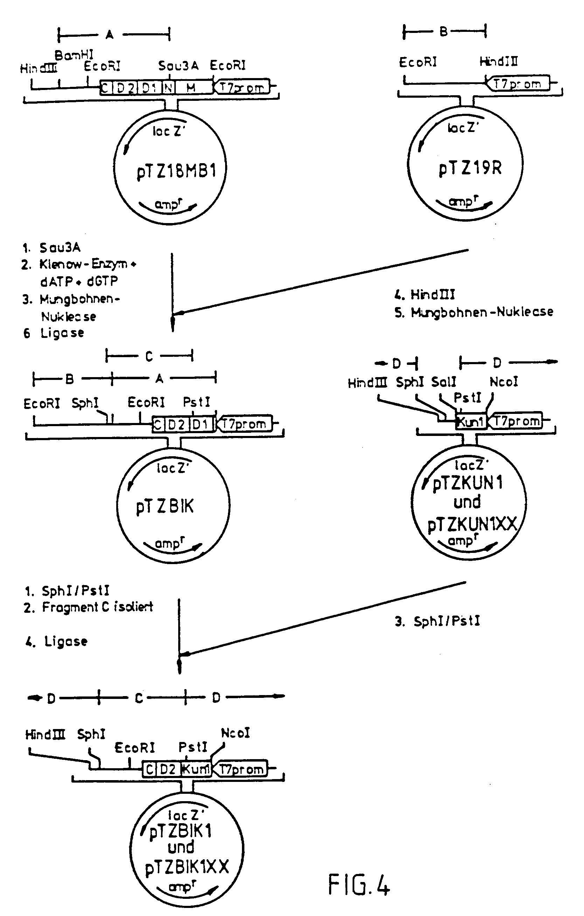

Plasmid pTZBIK wurde aus der in Plasmid pTZ18R klonierten cDNA eines $\alpha_1$-Mikroalobulin-Bikunin-Klons (pTZ18MB1) durch Spaltung mit der Restriktionsnuklease Sau3A erhalten. Dabei wurde Fragment A (Abb.4) erzeugt und gelelektrophoretisch isoliert. Das Fragment besitzt die nahezu vollständige Sequenz des Bikunin-Gens ohne den $\alpha_1$-Mikroglobulin-kodierenden Anteil und einen kleinen Teil des Genabschnitts für das N-terminale Peptid des Bikunins. Die Fragmentenden wurden mit dATP und dGTP in Gegenwart von Klenow-Enzym selektiv aufgefüllt, mit Mungbohnen-Nuklease geglättet und in Plasmid pTZ19R (Pharmacia) kloniert, das mit HindIII linearisiert worden war und dessen Enden ebenfalls mit Mungbohnen-Nuklease geglättet worden waren. Transformiert wurde in E. coli DH5. Die Plasmid-DNA der erhaltenen Klone wurde durch Sequenzanalyse verifiziert.

Die Plasmide pTZBIK1 und pTZBIK1XX (XX = IL, LM, MM, LL, VL) wurden durch Ersatz des natürlichen Genabschnitts für die Domäne 1 des Bikunin-Gens durch den entsprechenden synthetischen Genabschnitt oder eine seiner Varianten erzeugt. Das nach Spaltung mit SphI und PstI isolierte Fragment C der pTZBIK-DNA, das die vollständige cDNA-Sequenz des Bikunins mit Ausnahme des Genabschnitts für nahezu die gesamte Domäne 1 enthält, wurde in entsprechend gespaltene Plasmide pTZKUN1 bzw. pTZKUN1XX (XX = IL, LM, MM, LL, VL) (siehe Abb. 3) ligiert und in E. coli DH5 kloniert. die Plasmid-DNA der erhalten Klone wurde durch Sequenzanalyse charakterisiert.

Konstruktion des des$[^{1-79}]$-Xaa$^{92}$-Xaa$^{94}$-Bikunin-Gens mit Xaa = Ile, Leu, Met, Val, Phe

Das Gen der 2. Domäne (des$[^{1-79}]$-Bikunin) wurde durch Verkürzen des Bikunin-Gens erhalten (Abb.5). Seine Varianten (des$[^{1-79}]$-Xaa$^{92}$-Xaa$^{94}$-Bikunin) wurden durch Austausch eines Apal/XmnI-Restriktionsfragments der Domäne 2 (Modul 3) mit geeigneten Kombinationen der Oligonukleotide A-D bzw. ihren Varianten (Abb.6) entsprechend Abb. 7 hergestellt.

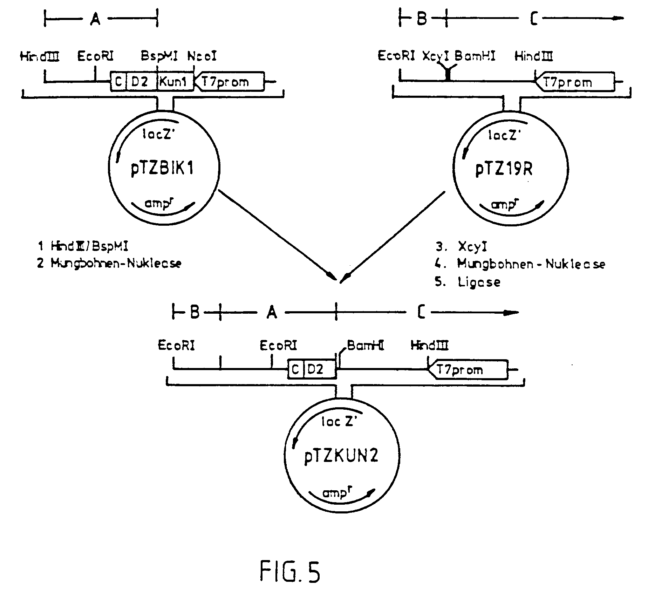

Plasmid pTZKUN2 (Abb.5) wurde durch Ligieren von Fragment A, das durch Behandlung von pTZBIK1-DNA mit den Restriktionsnukleasen HindIII und BspMI erzeugt und an den Fragmentenden mit Mungbohnen-Nuklease geglättet worden war, mit XcyI-gespaltenem und ebenfalls mit Mungbohnen-Nuklease behandeltem Vektor pTZ19R (Pharmacia) nach Transformieren von E. coli DH5-Zellen erhalten. Die Plasmid-DNA einzelner Klone wurde durch Sequenzanalyse charakterisiert.

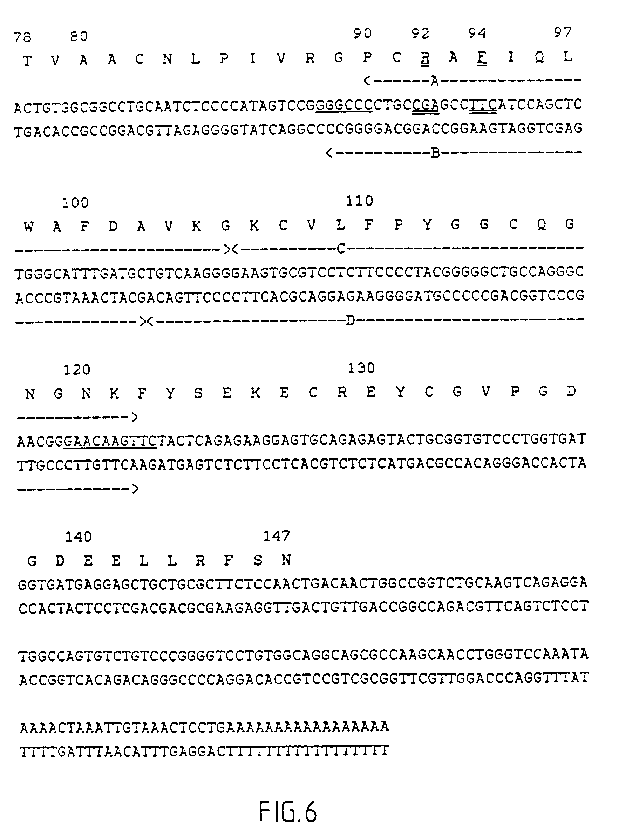

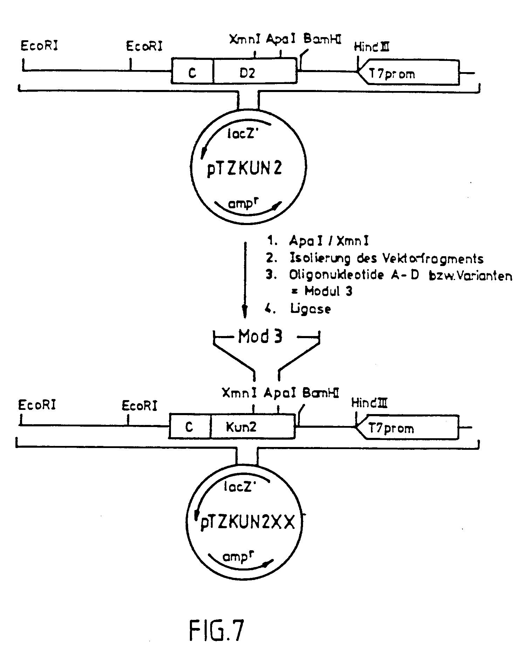

Die Plasmide pTZKUN2XX (XX = IL, LL, VL und LF) wurden durch Ersatz eines Apal/XmnI-Fragments von pTZKUN2 durch die entsprechenden Oligonukleotide des Moduls 3 (siehe Abb. 6) erhalten. Das Vektorfragment wurde nach Spaltung mit den genannten Restriktionsnukleasen und Dephosphorylierung der 5′-Enden mit alkalischer Phosphatase aus Kälberdarm isoliert. Nach Ligierung mit den mit Polynukleotidkinase phosphorylierten Oligonukleotiden des Moduls 3 und Transformation in E. coli DH5 wurde die Plasmid-DNA einzelner Klone durch Kartierung und schließlich Sequenzanalyse charakterisiert (Abb.7).

Konstruktion von des$[^{1-17}]$-Met$^{18}$-Xaa$^{36}$-Xaa$^{38}$-Xaa$^{92}$-Xaa$^{94}$-Bikunin-Genen

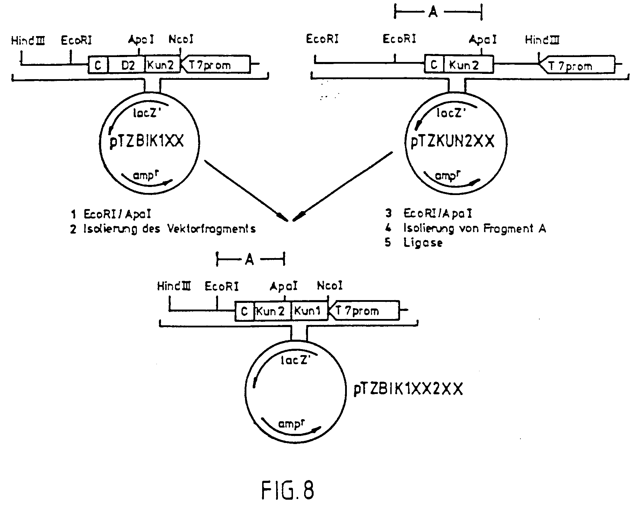

Genvarianten des Bikunins mit Austauschen in beiden Domänen wurden aus den Bikunin-Genvarianten mit varianter Domäne 1 (pTZBIK1XX; Abb.4) durch Ersatz eines Genabschnitts der natürlichen Domäne 2 durch einen entsprechenden Abschnitt der Genvarianten der Domäne 2 (Abb.7) auf dem in Abb.8 gezeigten Weg erzeugt.

Nach Spaltung von Plasmid pTZBIXX durch die Restriktionsnukleasen EcoRI und Apal, Dephosphorylierung der 5′-Enden der DNA-Fragmente mit alkalischer Phosphatase aus Kälberdarm und Isolierung des Vektorfragments wurde mit dem ebenfalls isolierten EcoRI/Apal-Fragment aus pTZKUN2XX ligiert und in E. coli DH5 kloniert. Die Plasmide einzelner Klone wurden durch Sequenzanalyse charakterisiert.

Konstruktion des des$[^{1-17,82-147}]$-Met$^{18}$-Xaa$^{36}$-Xaa$^{38}$-Bikunin-Gens

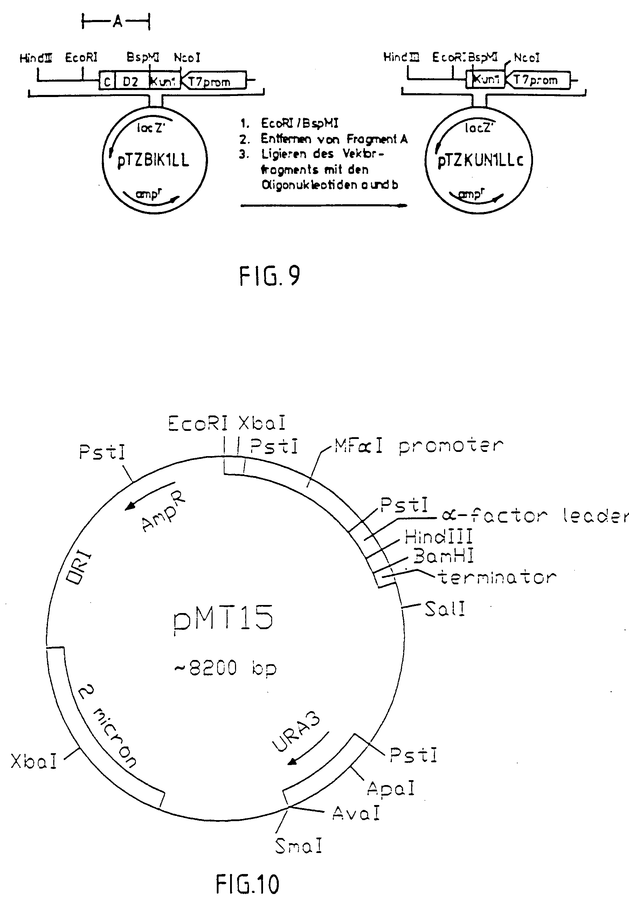

Gene der 1. Domäne mit zusätzlicher C-terminaler Verlängerung um die Aminosäuresequenz T-V-A-A (des$[^{1-17,82-147}]$-Met$^{18}$-Xaa$^{36}$-Xaa$^{38}$-Bikunin) wurden aus den Plasmiden pTZBIK1XX der entsprechenden Bikuninvarianten nach Ersatz der 2. Domäne durch die Oligonukleotide a (5′-CTGTGGCGGCCTGAG-3′) und b (5′-AATTCTCAGGCCGCC-3′) erhalten (Abb.9). Dazu wurde pTZBIK1XX mit BspMI und EcoRI gespalten, die 5′-Enden der DNA-Fragmente mit alkalischer Phosphatase aus Kälberdarm dephosphoryliert, das Vektorfragment isoliert und in Gegenwart der Oligonukleotide ligiert. Nach Transformation von E. coli DH5 wurden die Plasmide pTZKUN1XXc einzelner Klone durch Sequenzanalyse verifiziert.

Expression von Bikunin-Varianten

Die beschriebenen Bikunin-Genvarianten konnten teilweise sowohl im Klonierungsvektor selbst als auch nach Umklonieren in verschiedene Expressionsvektoren wie z.B. pJLA502 (Schauder et al., Gene 52, 279-283, 1987), pEX3 (Stanley & Luzio, EMBO J. 3, 1429-1434, 1984) und pEX3407 (Kocken et al. FEBS Lett. 236, 132-134, 1988) exprimiert werden. Beispielsweise lassen sich NcoI/SalI-bzw. NcoI/EcoRI-Fragmente aus den Plasmiden pTZKUN1XX bzw. pTZBIK1XX2XX (Abb. 3 und 8) isolieren und direkt in entsprechend geschnittenen, mit alkalischer Phosphatase aus Kälberdarm behandelten Vektor pJLA502 ligieren. Alternativ lassen sich Fragmente aus den Plasmiden pTZBIK1XX (EcoI/EcoRI-Fragment, Abb. 4), pTZBIK1XX2XX (NcoI/EcoRI-Fragment, Abb.8), pTZKUN1XX (NcoI/HindIII-Fragment, Abb.3) isolieren, mit Mungbohnen-Nuklease glätten und in die Vektoren pEX3 oder pEX3407 ligieren.

Fragmente des Plasmids pTZKUN2XX (Abb.7) lassen sich durch Spalten mit EcoRI, Behandlung mit Mungbohnen-Nuklease und Nachspalten mit BamHI erzeugen und in die pEX-Vektoren ligieren. DNA der pEX-Vektoren kann dazu entweder mit SalI geschnitten und mit Mungbohnen-Nuklease behandelt werden oder, wie in der zuletzt genannten Konstruktion, mit PstI gespalten, mit Mungbohnen-Nuklease behandelt und mit BamHI nachgespalten werden. Transformanten von E. coli pop2136 (Vidal-Ingigliardi & Raibaud, Nucleic Acids Res. 13, 1163, 1985) erlauben eine temperaturabhängige Kontrolle der Genexpression. In allen Fällen entstehen Fusionsproteine, die über eine säurelabile Asp-Pro-Peptidbindung mit dem zu exprimierenden Protein verknüpft sind. Nach Abtrennen des Fusionsproteinanteils, Reinigung und Renaturierung des Bikuninanteils resultierten Inhibitorvarianten des Typs des[$^{1-17}$]-Pro$^{18}$-Bikunin, des[$^{1-17,78-147}$]-Pro$^{18}$-Bikunin und des[$^{1-78}$]-Pro$^{79}$-Bikunin. Die Spaltung des Bikuninteils vom Fusionspartner kann auch durch Verwendung der proteolytisch (tryptisch) besonders labilen Sequenz Pro$^{20}$-Lys$^{21}$-Lys$^{22}$ erzielt werden. Einzelköpfige Varianten konnten besonders gut in sekretorischen Expressionssystemen exprimiert werden.

Der Nachweis der Expressionsprodukte erfolgte entweder durch Gelelektrophorese (z.B. zur Bestimmung von Fusionsproteinen) oder in Standard-Enzymhemmtesten (nach Abspaltung des Fusionspartners, Reinigung und Renaturierung des Bikuninteils).

Die erzielten Ergebnisse zeigten, daß z.B. des[$^{1-17}$]-Met-18-Leu-36-Leu-38-Bikunin, des[$^{1-17}$]-Met-18-Leu-36-Leu-38-Leu-92-Leu-94-Bikunin und des[$^{1-17}$]-Met-18-Leu-92-Leu-94-Bikunin, hergestellt in der beschriebenen Weise durch Expresion in E. coli, potente Protease-hemmende Wirkung aufwiesen.

Beispiel 2

Expression von Leu-36-Leu-38-Bikunin

Klonierung des Gens: pCY17, ein von pBR322 abgeleiteter Vektor, der das MAT-alpha-1-Strukturgen enthält (Kurjan und Herskowitz, Cell, 30, 933, 1982) wurde von Herskowitz, University of California, erhalten. pMT15 ist ein Hefe-E. coli-Shuttle-Vektor (Abb. 10). Er trägt Amp$^R$, bla und URA3 Gen-Segmente, die als selektierbare Marker für E. coli und Hefe dienen. pMT15 besitzt außerdem den ColE1-Origin von pBR322 und ein Segment der B-Form des 2 u Plasmids, so daß das Plasmid sowohl in E. coli als auch in Hefe stabil repliziert werden kann. Es trägt außerdem dem MAT1-alpha-Promotor und die kodierende Sequenz der N-terminalen Prä-Pro-Sequenz des alpha-Faktor-Vorläuferproteins als EcoRI-HindIII-Fragment, das aus dem Plasmid pCY17 erhalten worden war. Diesem Fragment folgte 3′-seitig ein 115 bp HindIII-BamHI-Fragment, das für die 34 N-terminalen Aminosäuren der Prä-Invertase kodiert (Das et al., Mol. Gen. Genet., 218, 240, 1989). Schließlich enthielt pMT15 am 3′-Ende der BamHI-Schnittstelle ein 160 bp Fragment eines Hefe-Transkriptionsterminators, der aus dem Hefe-URA3-Gen erhalten worden war (Yarger et al. Mol. Cell. Biol. 8, 1095, 1986).

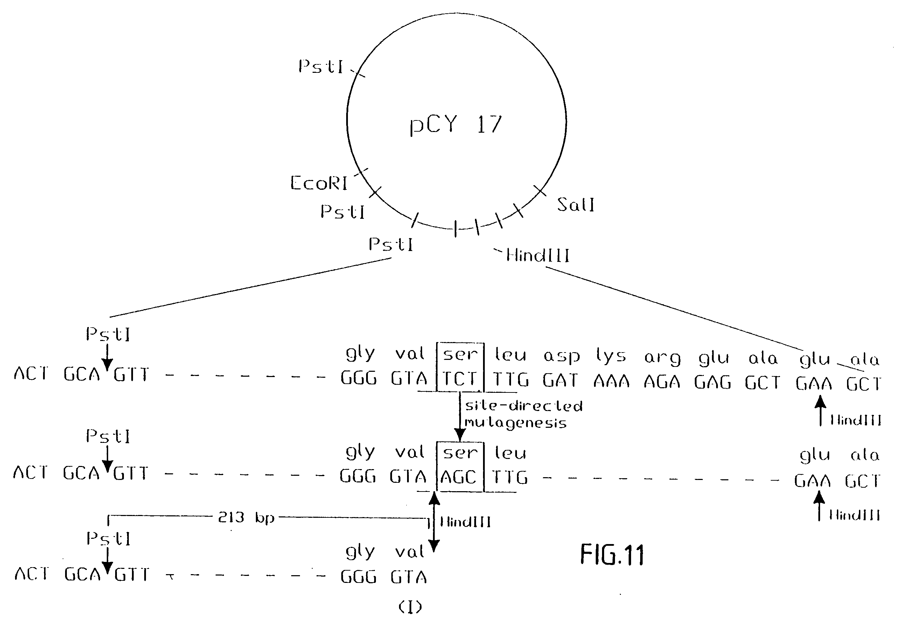

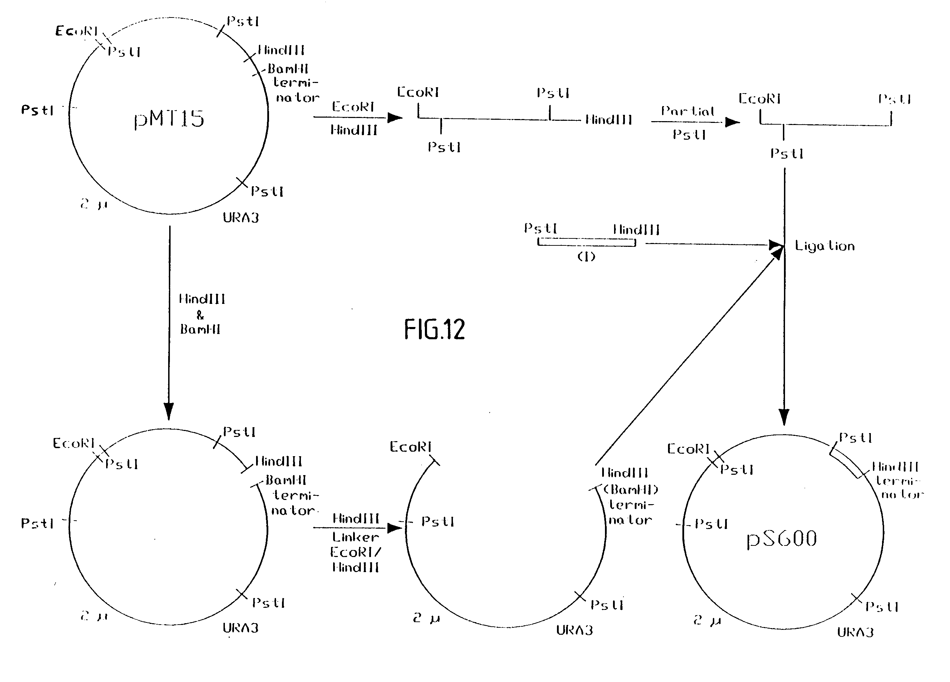

Das 235 bp PstI-HindIII-Fragment von pMT15, das die kodierende Region der alpha-Faktor-Prä-Pro-Leadersequenz trägt, wurde in den Vektor M13mp18 kloniert und einer gezielten Mutagenese unterworfen unter Verwendung des mutagenen Oligonukleotids 5′-GAA GAA GGG GTA TTG GAT AAA AGA-3′. Als Ergebnis der Mutagenese wurde das Serin-Kodon bei Position 81 in der alpha-Faktor-Prä-Pro-Sequenz von TCT zu AGC geändert, wodurch eine HindIII-Restriktionsschnittstelle erzeugt wurde. Mit dem dadurch hergestellten 213 bp PstI-HindIII-Fragment (Abb. 11) wurde in pMT15 das 235 bp PstI-HindIII-Fragment ersetzt. Das so modifizierte Plasmid wurde mit pS600 bezeichnet; es trägt die kodierende Sequenz für Lys-Arg statt Lys-Arg-Glu-Ala-Glu-Ala als Prozessierungsstelle (Abb. 12).

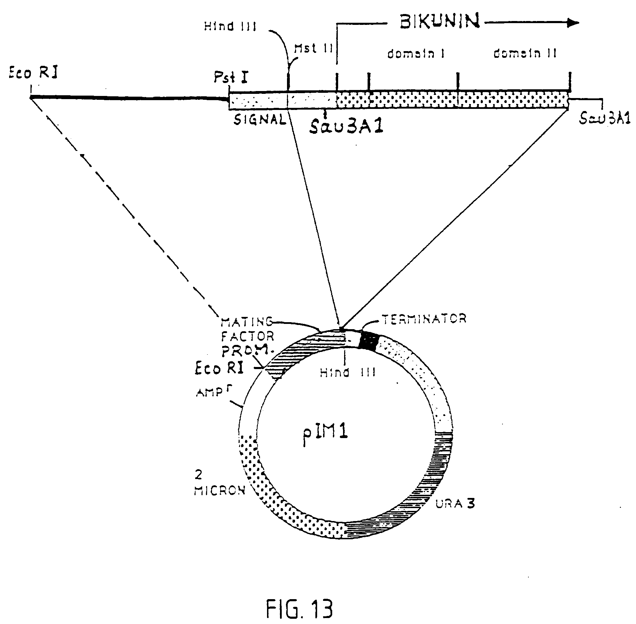

Konstruktion des Leu-36-Leu-38-Bikunin-Gens: Ein 540 bp Sau3AI-Fragment aus dem Plasmid PIM1 (Das und Lehman, J. Cell. Biochem, 2B, 284, 1988), das die kodierende Sequenz des Bikunin trägt (Abb. 13), wurde in M13mp19 kloniert und einer gezielten Mutagenese unterworfen, um Met-36 in Leu-36 und Met-38 in Leu-38 umzuwandeln. Die Mutagenese wurde nach der Methode von Sayers et al. (Nucl. Acids. Res., 16, 79, (1988) durchgeführt. Ferner wurde ein Doppel-Strang-DNA-Fragment der folgenden Sequenz hergestellt:

```
5'...AGC TTG GAT AAA AGA GCT GTG CTA CCC CAA

3'-      AC CTA TTT TCT CGA CAC GAT GGG GTT


GAA GAG GAA G..........3'

CTT CTC CTT CCT AG ....5'
```

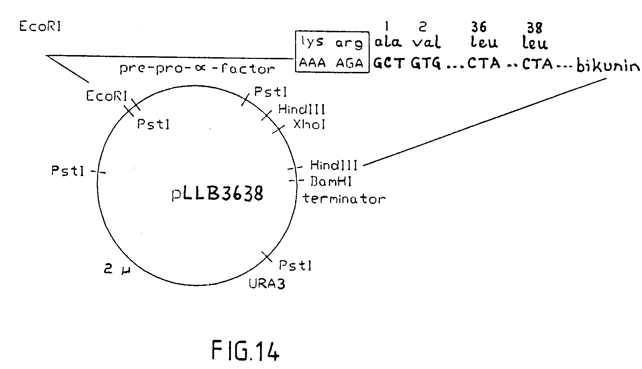

Das DNA-Fragment wurde nach Standardverfahren phosphoryliert. Das so erhaltene DNA-Fragment wurde zusammen mit der mutierten Bikunin-Sequenz in das mit HindIII und BamHI verdaute 8,2 bp pS600 Plasmid eingebaut. Das Oligomer rekonstituierte sowohl das 3'-Ende der Prä-Pro-alpha-Faktor-Sequenz als auch das 5'-Ende der Bikunin-Sequenz. Das so erhaltene neue Plasmid pLLB3638 enthält demnach die kodierende Region für die Prä-Pro-alpha-Faktor-Sequenz mit Lys-Arg als Prozessierungsstelle fusioniert an die Leu-36-Leu-38-Bikunin-Sequenz. Die DNA und die entsprechende Aminosäuresequenz an der Fusionsstelle von Prä-Pro-alpha-Faktor-Leu-36-Leu-38-Bikunin in pLLB3638 ist in der Abb. 14 dargestellt.

Expression, Sekretion und Nachweis des inhibitorisch aktiven Leu-36-Leu-38-Bikunins: Entsprechend der oben beschriebenen Methoden wurde die Hefezellinie SC 106 (MAT, hom3, gal2, his6, ura3) mit pLLB3638 transformiert; URA3$^+$-Zellen wurden isoliert und kultiviert; der Kulturüberstand wurde auf Elastase-inhibitorische Aktivität überprüft. In Kontrollversuchen wurde nachgewiesen, daß SC106-Zellen transformiert mit pS600 keine Elastase-inhibitorische Aktivität im Kulturmedium produzierten.

Die erzielten Ergebnisse zeigen, daß das Leu-36-Leu-38-Bikunin-Gen, exprimiert in Saccharomyces cerevisiae, zur Bildung von zwei Proteinspezies im Kulturmedium mit den folgenden Eigenschaften führte: (a) Trysininhibitorisch aktiv, (b) Molekulargewicht von 20 k und 17 k nach Elektrophorese in SDS-Polyacrylamidgelen, (c) von jeder Proteinspezies zeigten ca. 60 % die N-terminale Sequenz des Bikunins und ca. 40 % die Sequenz des des(Ala-1)-Bikunins.

Beispiel 3

Expression von Leu-92-Leu-94-Bikunin

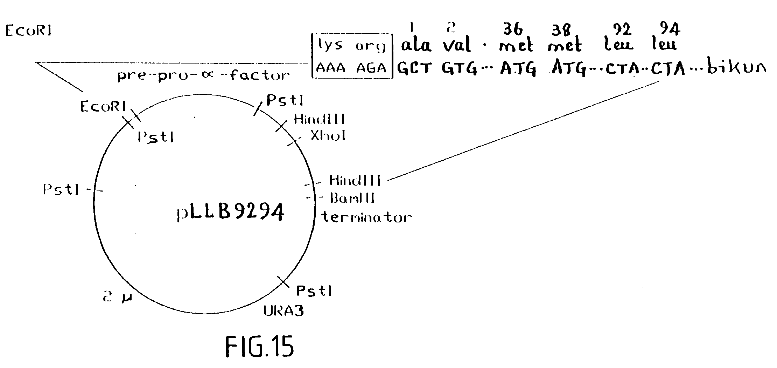

Klonierung des Gens: Das 540 bp Sau3AI-Fragment der pIM1-DNA (Das und Lehman, J. Cell. Biochem. 12 B, 284, 1988) mit der kodierenden Sequenz des Bikunins (Abb. 13) wurde in das Plasmid M13mp19 kloniert und einer gezielten Mutagenese unterworfen, um Arg-92 zu Leu-92 und Phe-94 zu Leu-94 zu ändern. Das so mutierte Fragment wurde analog zu Beispiel 1 in das Plasmid pS600 kloniert. Die DNA- und die entsprechende Aminosäure-Sequenz an der Fusionsstelle des Prä-Pro-alpha-Faktor-Leu-92-Leu-94-Bikunin-Gens im Expressionsvektor pLLB9294 ist in Abb 15 dargestellt.

Expression, Sekretion und Nachweis des inhibitorisch aktiven Leu-92-Leu-94-Bikunins: Entsprechend der oben beschriebenen Methoden wurde SC106 mit pLLB9294 transformiert; URA3$^+$-Zellen wurden isoliert und kultiviert; der Kulturüberstand wurde auf Elastase-inhibitorische Aktivität überprüft. In Kontrollversuchen wurde nachgewiesen, daß SC106-Zellen transformiert mit pS600 keine Elastase-inhibitorische Aktivität im Kulturmedium produzierten.

Die erzielten Ergebnisse zeigen, daß das Leu-92-Leu-94-Bikunin-Gen, experimiert in saccharomyces cerevisiae, zur Bildung von zwei Proteinspezies im Kulturmedium mit den folgenden Eigenschaften führte: (a) Elastaseinhibitorisch aktiv, (b) Molekulargewicht von 20 k und 17 k nach Elektrophorese in SDS-Polyacrylamidgelen, (c) von jeder Proteinspezies zeigten ca. 60 % die N-terminale Sequenz des Bikunins und ca. 40 % die Sequenz des des (Ala-1)-Bikunins.

Beispiel 4

Expression von Leu-36-Leu-38-Leu-92-Leu-94-Bikunin

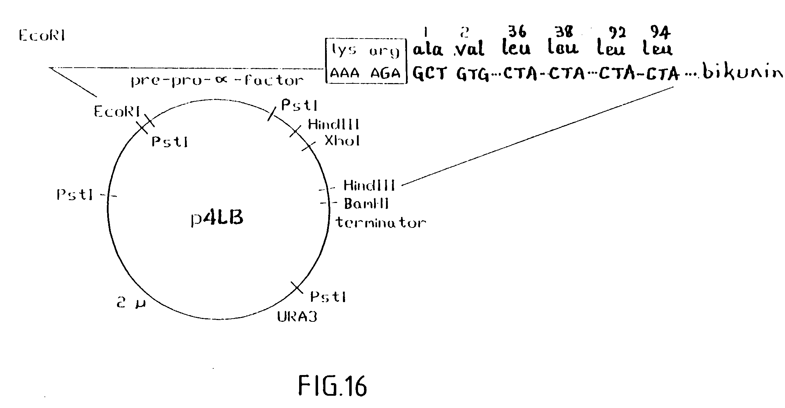

Klonierung des Gens: Das 540 bp Sau3Al-Fragment der pIM1-DNA (Das und Lehmann, J. Cell. Biochem 12 B, 284, 1988) mit der kodierenden Sequenz des Bikunins (Abb. 13) wurde in das Plasmid M13mp19 kloniert und einer gezielten Mutagenese unterworfen, um Met-36 zu Leu-36, Met-38 zu Leu-38, Arg-92 zu Leu-92 und Phe-94 zu Leu-94 zu ändern. Das so mutierte Fragment wurde analog zu Beispiel 1 in das Plasmid pS600 kloniert. Die DNA- und die entsprechende Aminosäure-Sequenz an der Fusionsstelle des Prä-Pro-alpha-Faktor-Leu-36-Leu-38-Leu-92-Leu-94-Bikunin-Gens im Expressionsvektor p4LB ist in Abb. 16 dargestellt.

Expression, Sekretion und Nachweis des inhibitorisch aktiven Leu-36-Leu-38-Leu-92-Leu-94-Bikunins: Entsprechend der oben beschriebenen Methoden wurde SC106 mit p4LB transformiert; URA3[+]-Zellen wurden isoliert und kultiviert; der Kulturüberstand wurde auf Elastase-inhibitorische Aktivität überprüft. In Kontrollversuchen wurde nachgewiesen, daß SC106-Zellen transformiert mit pS600 keine Elastase-inhibitorische Aktivität im Kulturmedium produzierten.

Die erzielten Ergebnisse zeigten, daß das Leu-36-Leu-38-Leu-92-Leu-94-Bikunin-Gen, exprimiert in saccharomyces cerevisiae, zur Bildung von zwei Proteinspezies im Kulturmedium mit den folgenden Eigenschaften führte: (a) Elastase-inhibitorisch aktiv, (b) Molekulargewicht von 20 k und 17 k nach Elektrophorese in SDS-Polyacrylamidgelen, (c) von jeder Proteinspezies zeigten ca. 60 % die N-terminale Sequenz des Bikunins und ca. 40 % die Sequenz des des(Ala-1)-Bikunins.

Beispiel 5

Expression von Des(1-21)-Leu-36-Leu-38-Bikunin

Klonierung des Gens: ein 480 bp PvuII-Fragment der pIM1-DNA (Das und Lehmann, J. Cell. Biochem. 12 B, 284, 1988) das die kodierende Region des Des(1-21)-Bikunin trägt (Abb. 13), wurde in das Plasmid M13mp19 kloniert und einer gezielten Mutagenese unterworfen, um Met-36 zu Leu-36 und Met-38 zu Leu-38 zu ändern. Die Mutagenese wurde nach der Methode von Sayers et al. (Nucl. Acids. Res., 16 791, 1988) durchgeführt. Ferner wurde ein Doppel-Strang-DNA-Fragment der folgenden Sequenz hergestellt:

```
5'-AGC TTG GAT AAA AGA AAA GAA GAT TCC TGC CAG-3'
     AC CTA TTT TCT TTT CTT CTA AGG ACG GTC-5'
```

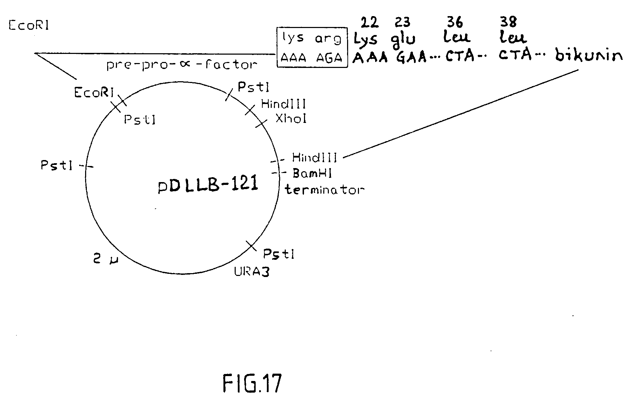

Das DNA-Fragment wurde nach Standardverfahren phosphoryliert. Das so erhaltene DNA-Fragment wurde zusammen mit der mutierten Bikunin-Sequenz in das mit HindIII und BamHI verdaute 8,2 bp pS600 Plasmid eingebaut. Das Oligomer rekonstituierte sowohl das 3'-Ende der Prä-Pro-alpha-Faktor-Sequenz als auch das 5'-Ende der Bikunin-Sequenz. Das so erhaltene neue Plasmid pDLLB-121 enthält demnach die kodierende Region für die Prä-Pro-alpha-Faktor-Sequenz mit Lys-Arg als Prozessierungsstelle fusioniert an die Des(1-21)-Leu-36-Leu-38-Bikunin-Sequenz. Die DNA und die entsprechende Aminosäuresequenz an der Fusionsstelle von Prä-Pro-alpha-Faktor-Leu-36-Leu-38-Bikunin in pDLLB-121 ist in der Abb. 17 dargestellt.

Expression, Sekretion und Nachweis des inhibitorisch aktiven Des(1-21)-Leu-36-Leu-38-Bikunins: Entsprechend der oben beschriebenen Methoden wurde SC106 mit pDLLB-121 transformiert; URA3[+]-Zellen wurden isoliert und kultiviert; der Kulturüberstand wurde auf Elastase-inhibitorische Aktivität überprüft. In Kontrollversuchen wurde nachgewiesen, daß SC106-Zellen transformiert mit pS600 keine Elastase-inhibitorische Aktivität im Kulturmedium produzierten.

Die erzielten Ergebnisse zeigten, daß das Des(1-21)-Leu-36-Leu-38-Bikunin-Gen, exprimiert in Saccharomyces cerevisiae, zur Bildung eines Proteins im Kulturmedium mit den folgenden Eigenschaften führte: (a) Trysininhibitorisch aktiv, (b) Molekulargewicht von 17 k und 15 k nach Elektrophorese in SDS-Polyacrylamidgelen, (c) das Protein zeigte die N-terminale Sequenz Lys-Glu-Asp-Ser-Cys des Des(1-21)-Bikunins.

Legenden

Abb. 1 Primärstruktur des menschlichen Bikunins Das N-terminale Peptid (Pos. 1-21) und die Domänen 1 (Pos. 22-77) bzw. 2 (Pos. 78-147) sind durch tryptische Spaltung erhältlich. Pfeile markieren Sequenzen solcher Bereiche, die von unterschiedlichen Exons kodiert werden, Sternchen die gezielt veränderten Reste $P_1$ und $P_2'$ der reaktiven Zentren. Identische Aminosäurereste in den beiden Inhibitordomänen des Bikunins sind durch vertikale Linien hervorgehoben.

Abb. 2 Konstruktion von des$[^{1-17, \, 78-147}]$-Met$^{18}$-Xaa$^{36}$-Xaa$^{38}$-Bikunin-Varianten. Oligonukleotidsequenz des Grundgens.

Die in den Varianten ausgetauschten Reste sind doppelt unterstrichen. Am 5'-Terminus des Gens ist das erste Kodon Teil der NcoI-Erkennungssequenz CCATGG, am 3'-Terminus des Gens ist das letzte Nukleotid des dritten von drei aufeinanderfolgenden Stoppkodons (end) Teil einer SalI-Erkennungssequenz GTCGAC. Die Erkennungssequenzen für die Restriktionsnukleasen NcoI, Asp718 und SalI, die die einzelnen Module begrenzen, sind einfach unterstrichen. Die zur Genkonstruktion verwendeten Oligonukleotide werden durch Pfeile repräsentiert und sind numeriert.

Abb.3 Klonierung des des$[^{1-17,78-147}]$-Met$^{18}$-Bikunin-Gens (pTZKUN1) und der des $[^{1-17,78-147}]$-Met$^{18}$-Xaa$^{36}$-Xaa$^{38}$-Bikunin-Genvarianten (pTZKUN1XX)

Abb.4 Klonierung des des$[^{1-17}]$-Bikunin-Gens (pTZBIK) bzw. des des$[^{1-17}]$-Met$^{18}$-Bikunin (pTZBIK1) und des$[^{1-17}]$-Met-$^{18}$-Xaa$^{36}$-Xaa$^{38}$-Bikunin-Gens (pTZBIK1XX).

M: $\alpha_1$-Mikroglobulin; D1 und D2: Domänen 1 und 2 des Bikunins; N: N-terminales Peptid des Bikunins; C: C-terminale Sequenz des Bikunins; KUN1: synthetische Domäne 1, bestehend aus den Modulen Mod1 und Mod2. Spaltstellen, die durch die Klonierungsprozedur verloren gehen, sind im resultierenden Plasmid eingetragen, jedoch nicht mehr gekennzeichnet. Die Fragmente B und D - wenn sie als solche nicht erzeugt wurden - sind zur besseren Orientierung angegeben.

Abb.5 Klonieren des des$[^{1-79}]$-Bikunin-Gens (pTZKUN2)

D2: Domäne 2 des Bikunins; C: C-terminale Sequenz des Bikunins; KUN1: synthetische Domäne 1, bestehend aus den Modulen Mod1 und Mod2: Spaltstellen, die durch die Klonierungsprozedur verloren gehen, sind im resultierenden Plasmid ohne Kennzeichnung eingetragen. Fragmente - auch wenn sie als solche nicht erzeugt wurden, wie z.B. B und C - werden zur besseren Orientierung angegeben

Abb. 6 Konstruktion der Genvarianten der 2. Domäne des Bikunins: des$[^{1-79}]$-Xaa$^{92}$-Xaa$^{94}$-Bikunin-Varianten und des$[^{1-17}]$-Met$^{18}$-Leu$^{36}$-Leu$^{38}$-Leu$^{92}$-Leu$^{94}$-Bikunin mit Xaa$^{92}$-Xaa$^{94}$ = Ile, Leu, Met, Val, Phe

Dargestellt ist die natürliche Bikuninsequenz ab Domäne 2 (Position 78) und die synthetischen Oligonukleotide A-D (Pfeile) in denen die Kodons der Positionen 92 und 94 des Bikunins (doppelt unterstrichen) in den Oligonukleotiden A und B jeweils entsprechend den Erfordernissen der einzelnen Varianten ersetzt wurden (Ile: ATC; Leu: CTG; Val: GTT; bzw. deren komplementäre Sequenzen im Gegenstrang). Die Erkennungssequenzen der Restriktionsnukleasen ApaI (GGGCCC) und XmnI (GAANNNNTTC) sind einfach unterstrichen.

Abb.7 Klonieren der des$[^{1-79}]$-Xaa$^{92}$-Xaa$^{94}$-Bikunin-Genvarianten mit Xaa = Ile, Leu, Met und Val

D2: Domäne 2 des Bikunins: KUN2: Domäne 2 des Bikunins nach Ersatz des natürlichen XmnI/ApaI-Fragments durch das synthetische Modul 3; C: C-terminale Sequenz des Bikunins

Abb.8 Klonieren der des$[^{1-17}]$-Met$^{18}$-Xaa$^{36}$-Xaa$^{38}$-Xaa$^{92}$-Xaa$^{94}$-Bikunin-Genvarianten mit Xaa = Ile, Leu, Met, Val, Phe

KUN1: synthetische Domäne 1, bestehend aus den Modulen 1 und 2;

KUN2: Domäne 2 des Bikunins nach Ersatz des natürlichen XmnI/ApaI-Fragments durch das synthetische Modul 3; C: C-terminale Sequenz des Bikunins

Abb.9 Klonieren des des$[^{1-17,82-147}]$-Met$^{18}$-Xaa$^{36}$-Xaa$^{38}$-Bikunin-Gens (C-terminale Verlängerung der 1. Domäne um die Aminosäuresequenz T-V-A-A)

KUN1: synthetische Domäne 1, bestehend aus den Modulen 1 und 2; D2: Domäne des Bikunins; C: C-terminale Sequenz des Bikunins; a und b: Oligonukletidlinker (siehe Text).

Abb. 10: pMT15, ein E. coli-Hefe-Shuttle-Vektor.

Abb. 11: Schema der gezielten Mutagenese der alpha-Faktor-Leader-Sequenz. Die Mutagenese von TCT zu AGC erleichterte die Isolierung eines 213 bp PstI-HindIII-Fragments, das mit (I) bezeichnet ist.

Abb. 12: Konstruktion eines modifizierten Hefe-E. coli-Shuttle-Vektors für die Expression/Sekretion des Bikunins unter Verwendung des alpha-Faktor-Promotors und der modifizierten alpha-Faktor-Leader-Sequenz. Die modifizierte alpha-Faktor-Leader-Sequenz (bezeichnet mit I in Abb. 11) wurde als PstI-HindIII-Gen-Fragment in den Vektor pMT15 eingebaut und ersetzte dort die ursprüngliche alpha-Faktor-Leader-Sequenz.

Abb. 13: Das natürlich vorkommende Bikunin-Gen kloniert in den pMT15 E. coli-Hefe-Shuttle-Vektor, der die alpha-Faktor-Prä-Pro-Sequenz enthält.

Abb. 14: Expressionsvektor pLLB3638. Prä-Pro-alpha-Faktor-Leu-36-Leu-38-Bikunin-Gen-Fusion kloniert in den Vektor pS600. DNA- und entsprechende Aminosäure-Sequenz an der Fusionsstelle von Prä-Pro-alpha-Faktor-Leu-36-Leu-38-Bikunin in pLLB3638 ist dargestellt. Lys-Arg ist die KEX2-Prozessierungsstelle der Prä-Pro-alpha-Faktor-Sequenz (Julius et al., Cell. 37, 1075, 1984). Die Aminosäure-Nummerierung 1 - 38 entspricht jener der Bikunin-Mutein-Sequenz.

Abb. 15: Expressionsvektor pLLB9294. Prä-Pro-alpha-Faktor-Leu-92-Leu-94-Bikunin-Gen-Fusion kloniert in den Vektor pS600. DNA- und entsprechende Aminosäure-Sequenz an der Fusionsstelle von Prä-Pro-alpha-Faktor-Leu-92-Leu-94-Bikunin in pLLB3638 ist dargestellt. Lys-Arg ist die KEX2-Prozessierungsstelle der Prä-Pro-alpha-Faktor-Sequenz (Julius et al., Cell. 37, 1075, 1984). Die Aminosäure-Nummerierung 1 - 94 entspricht jener der Bikunin-Mutein-Sequenz.

Abb. 16: Expressionsvektor p4LB. Prä-Pro-alpha-Faktor-Leu-36-Leu-38-Leu-92-Leu-94-Bikunin-Gen-Fusion kloniert in den Vektor pS600. DNA- und entsprechende Aminosäure-Sequenz an der Fusionsstelle von Prä-Pro-alpha-Faktor-Leu-36-Leu-38-Leu-92-Leu-94-Bikunin in p4LB ist dargestellt Lys-Arg ist die KEX2-Prozessierungsstelle der Prä-Pro-alpha-Faktor-Sequenz (Julius et al., Cell 37, 1075, 1984). Die Aminosäure-Nummerierung 1 - 94 entspricht jener der Bikunin-Mutein-Sequenz.

Abb. 17: Expressionsvektor pDLLB-121. Prä-Pro-alpha-Faktor-Des(1-21)-Leu-36-Leu-38-Bikunin-Gen-Fusion kloniert in den Vektor pS600. DNA- und entsprechende Aminosäure-Sequenz an der Fusionsstelle von Prä-Pro-alpha-Faktor-Des(1-21)-Leu-36-Leu-38-Bikunin in pDLLB-121 ist dargestellt. Lys-Arg ist die KEX2-Prozessierungsstelle der Prä-Pro-alpha-Faktor-Sequenz (Julius et al., Cell. 37, 1075, 1984). Die Aminosäure-Nummerierung 1 - 38 entspricht jener der Bikunin-Mutein-Sequenz.

**Patentansprüche**

**Patentansprüche für folgenden Vertragsstaaten : AT, BE, CH, DE, DK, FR, GB, GR, IT, LI, LU, NL, SE**

1. Proteinaseinhibitor, der die Sequenz der Aminosäuren 21 bis 147 des humanen Bikunins besitzt mit beliebigen Kombinationen der folgenden Austausche:

   Position 36:     Met gegen Ile, Val, Arg, Phe, Tyr, Trp, Lys
   Position 38:     Met gegen Arg, Ile, Val, Lys
   Position 45:     Asn gegen eine andere natürliche Aminosäure
   Position 92:     Arg gegen Leu, Ile, Val, Phe, Lys
   Position 94:     Phe gegen Leu, Arg, Lys, Ile, Val
   Position 98:     Trp gegen Lys, Ile, Val, Phe, Leu, Ala, Gly, Ser

2. Proteinaseinhibitor gemäß Anspruch 1, bei dem ein oder mehrere Met-Reste durch nicht oxidierbare Aminosäurereste ersetzt sind.

3. Proteinaseinhibitor gemäß den Ansprüchen 1 bis 2 mit einem zusätzlichen Polypeptid am N-Terminus dessen Aminosäuresequenz von der natürlichen Sequenz 1 bis 21 des Bikunins abgeleitet ist.

4. Proteinaseinhibitor gemäß den Ansprüchen 1 bis 3 mit oder ohne Glykosylierung.

5. Fragmente mit Proteinasen-inhibotorischer Wirkung, die von den Proteinaseninhibitoren gemäß den Ansprüchen 1 bis 4 erhalten werden.

6. Fragmente gemäß Anspruch 5, die im wesentlichen die Sequenz der Aminosäuren 22 bis 77, 1 bis 77 oder 78 bis 147 des Bikunins enthalten.

7. Verfahren zur Herstellung von Proteinaseinhibitoren gemäß den Ansprüchen 1 bis 6, dadurch gekennzeichnet, daß man eine geeignete Wirtszelle, die mit einer für diesen Inhibitor codierenden Nucleinsäure verändert wurde, kultiviert und den Inhibitor aus der Kultur gewinnt.

8. Arzneimittel enthaltend einen oder mehrere der Proteinaseinhibitoren aus den Ansprüchen 1 bis 6.

9. Verwendung von Proteinaseinhibitoren aus den Ansprüchen 1 bis 6 zur Herstellung von Arzneimitteln.

EP 0 401 508 B1

**Patentansprüche für folgenden Vertragsstaat : ES**

1. Verfahren zur Herstellung von Proteinaseinhibitoren, die die Sequenz der Aminosäuren 21 bis 147 des humanen Bikunins besitzen mit beliebigen Kombinationen der folgenden Austausche:

   Position 36:    Met gegen Ile, Val, Arg, Phe, Tyr, Trp, Lys
   Position 38:    Met gegen Arg, Ile, Val, Lys
   Position 45:    Asn gegen eine andere natürliche Aminosäure
   Position 92:    Arg gegen Leu, Ile, Val, Phe, Lys
   Position 94:    Phe gegen Leu, Arg, Lys, Ile, Val
   Position 98:    Trp gegen Lys, Ile, Val, Phe, Leu, Ala, Gly, Ser

   dadurch gekennzeichnet, daß man eine geeignete Wirtszelle, die mit einer für diese Inhibitoren codierenden Nucleinsäure verändert, kultiviert und den Inhibitor aus der Kultur gewinnt.

2. Verfahren gemäß Anspruch 1, wobei bei den Proteinaseinhibitoren ein oder mehrere Met-Reste durch nicht oxidierbare Aminosäurereste ersetzt sind.

3. Verfahren gemäß den Ansprüchen 1 und 2 wobei die Proteiaseinhibitoren ein zusätzliches Polypeptid am N-Terminus tragen, dessen Aminosäure-Sequenz von der natürlichen Sequenz 1 bis 21 des Bikunins abgeleitet ist.

4. Verfahren gemäß den Ansprüchen 1 bis 3 zur Herstellung von Proteaseinhibitoren mit oder ohne Glykosylierung.

**Claims**

**Claims for the following Contracting States : AT, BE, CH, DE, DK, FR, GB, GR, IT, LI, LU, NL, SE**

1. Proteinase inhibitor which has the sequence of amino acids 21 to 147 of human bikunin, with any desired combinations of the following replacements:

   Position 36:    Met by Ile, Val, Arg, Phe, Tyr, Trp, Lys
   Position 38:    Met by Arg, Ile, Val, Lys
   Position 45:    Asn by another natural amino acid
   Position 92:    Arg by Leu, Ile, Val, Phe, Lys
   Position 94:    Phe by Leu, Arg, Lys, Ile, Val
   Position 98:    Trp by Lys, Ile, Val, Phe, Leu, Ala, Gly, Ser.

2. Proteinase inhibitor according to Claim 1, in which one or more Met residues are replaced by non-oxidizable amino acid residues.

3. Proteinase inhibitor according to Claims 1 and 2, with an additional polypeptide at the N-terminus whose amino acid sequence is derived from the natural sequence 1 to 21 of bikunin.

4. Proteinase inhibitor according to Claims 1 to 3, with or without glycosylation.

5. Fragments with proteinase-inhibitory action, which are obtained from the proteinase inhibitors according to Claims 1 to 4.

6. Fragments according to Claim 5, which essentially contain the sequence of amino acids 22 to 77, 1 to 77 or 78 to 147 of bikunin.

7. Process for the preparation of proteinase inhibitors according to Claims 1 to 6, characterized in that a suitable host cell which has been modified with a nucleic acid coding for this inhibitor is cultivated and the inhibitor is obtained from the culture.

8. Medicaments containing one or more of the proteinase inhibitors from Claims 1 to 6.

9. Use of proteinase inhibitors from Claims 1 to 6 for the production of medicaments.

14

EP 0 401 508 B1

**Claims for the following Contracting State: ES**

1. Process for the preparation of proteinase inhibitors which have the sequence of amino acids 21 to 147 of human bikunin, with any desired combinations of the following replacements:

   Position 36:   Met by Ile, Val, Arg, Phe, Tyr, Trp, Lys
   Position 38:   Met by Arg, Ile, Val, Lys
   Position 45:   Asn by another natural amino acid
   Position 92:   Arg by Leu, Ile, Val, Phe, Lys
   Position 94:   Phe by Leu, Arg, Lys, Ile, Val
   Position 98:   Trp by Lys, Ile, Val, Phe, Leu, Ala, Gly, Ser characterized in that a suitable host cell which has been modified with a nucleic acid coding for these inhibitors is cultivated and the inhibitor is obtained from the culture.

2. Process according to Claim 1, in which in the proteinase inhibitors one or more Met residues are replaced by non-oxidizable amino acid residues.

3. Process according to Claims 1 and 2, in which the proteinase inhibitors carry an additional polypeptide at the N-terminus whose amino acid sequence is derived from the natural sequence 1 to 21 of bikunin.

4. Process according to Claims 1 to 3 for the preparation of proteinase inhibitors with or without glycosylation.

**Revendications**
**Revendications pour les Etats contractants suivants : AT, BE, CH, DE, DK, FR, GB, GR, IT, LI, LU, NL, SE**

1. Inhibiteur de protéinase, qui possède la séquence d'amino-acides 21 à 147 de la bikunine humaine avec des combinaisons quelconques des échanges suivants :

   position 36 :   Met contre Ile, Val, Arg, Phe, Tyr, Trp, Lys
   position 38 :   Met contre Arg, Ile, Val, Lys
   position 45 :   Asn contre un autre amino-acide naturel
   position 92 :   Arg contre Leu, Ile, Val, Phe, Lys
   position 94 :   Phe contre Leu, Arg, Lys, Ile, Val
   position 98 :   Trp contre Lys, Ile, Val, Phe, Leu, Ala, Gly, Ser.

2. Inhibiteur de protéinase suivant la revendication 1, dans lequel un ou plusieurs restes Met sont remplacés par des restes d'amino-acides non oxydables.

3. Inhibiteur de protéinase suivant les revendications 1 et 2, comprenant un polypeptide supplémentaire à l'extrémité terminale N dont la séquence d'amino-acides est dérivée de la séquence naturelle 1 à 21 de la bikunine.

4. Inhibiteur de protéinase suivant les revendications 1 à 3, avec ou sans glycosylation.

5. Fragments à activité inhibitrice de protéinase, qui sont obtenus par les inhibiteurs de protéinase suivant les revendications 1 à 4.

6. Fragments suivant la revendication 5, qui contiennent principalement la séquence des amino-acides 22 à 77, 1 à 77 ou 78 à 147 de la bikunine.

7. Procédé de préparation d'inhibiteurs de protéinase suivant les revendications 1 à 6, caractérisé en ce qu'on cultive une cellule-hôte appropriée qui a été modifiée avec un acide nucléique codant pour cet inhibiteur et on isole l'inhibiteur de la culture.

8. Médicament contenant un ou plusieurs des inhibiteurs de protéinase suivant les revendications 1 à 6.

9. Utilisation d'inhibiteurs de protéinase suivant les revendications l à 6 pour la préparation de médicaments.

15

**Revendications pour l'Etat contractant suivant : ES**

1. Procédé de préparation d'inhibiteurs de protéinase, qui possèdent la séquence d'amino-acides 21 à 147 de la bikunine humaine avec des combinaisons quelconques des échanges suivants :

    position 36 :    Met contre Ile, Val, Arg, Phe, Tyr, Trp, Lys

    position 38 :    Met contre Arg, Ile, Val, Lys

    position 45 :    Asn contre un autre amino-acide naturel

    position 92 :    Arg contre Leu, Ile, Val, Phe, Lys

    position 94 :    Phe contre Leu, Arg, Lys, Ile, Val

    position 98 :    Trp contre Lys, Ile, Val, Phe, Leu, Ala, Gly, Ser.

    caractérisé en ce qu'on cultive une cellule-hôte appropriée qui est modifiée avec un acide nucléique codant pour ces inhibiteurs, et on isole l'inhibiteur de la culture.

2. Procédé suivant la revendication 1, dans lequel un ou plusieurs des restes Met dans les inhibiteurs de protéinase sont remplacés par des restes d'amino-acides non oxydables.

3. Procédé suivant les revendications 1 et 2, dans lequel les inhibiteurs de protéinase portent un polypeptide supplémentaire à l'extrémité terminale N dont la séquence d'amino-acides est dérivée de la séquence naturelle 1 à 21 de la bikunine.

4. Procédé suivant les revendications 1 à 3 pour la préparation d'inhibiteurs de protéinase avec ou sans glycosylation.

```
                                                      10              20
                                    A V L P G E E E G S G G G Q L V T E V T K

22            30       ǀ ǀ 40          50·            60              70        77
K E D S C Q L G Y S A G P C M G M T S R Y F Y N G T S M A C E T F Q Y G G C M G N G N N F V T E K E C L Q T C R
        ^

      ǀ ǀ      ǀǀǀ                        ǀ     ǀ  ǀǀǀǀ  ǀǀǀǀ ǀ  .ǀǀǀǀ       ǀ

78 80         90  ǀ ǀ    100           110           120             133
T V A A C N L P I V R G P C R A F I Q L W A F D A V K G K C V L F P Y G G C Q G N G N K F Y S E K E C R E Y C G
        ^

      140        147
V P G D G D E E L L R F S N
```

## FIG.1

```
NcoI          22                          30                  36
    M  V  T  K  K  E  D  S  C  Q  L  G  Y  S  A  G  P  C  M̲
    <-------------------------1-------------------------->X---
    CCATGGTAACTAAAAAGAAGACTCTTGCCAGCTGGGCTACTCTGCTGGTCCGTGCATGG  60
        CATTGATTTTTTCTTCTGAGAACGGTCGACCCGATGAGACGACCAGGCACGTACC
    <-------------4-----------X---------------5-------------


              Modul 1   < >   Modul 2
      38    40               Asp718      50
    G  M̲  T  S  R  Y  F  Y  N  G  T  S  M  A  C  E  T  F  Q  Y
    -2-----X---------3---------X------------------7-------------
    GTATGACTTCTCGTTACTTCTACAACGGTACCTCTATGGCTTGCGAAACTTTCCAGTACG  120
    CATACTGAAGAGCAATGAAGATGTTGCCATGGAGATACCGAACGCTTTGAAAGGTCATGC
    -X---------------6-------------X--------------------10-----


          60                        70
    G  G  C  M  G  N  G  N  N  F  V  T  E  K  E  C  L  Q  T  C
    --X------------------8-----------------X-----------------9----
    GTGGTTGCATGGGTAACGGTAACAACTTCGTTACTGAAAAAGAATGCCTGCAGACTTGCC  180
    CACCAACGTACCCATTGCCATTGTTGAAGCAATGACTTTTTCTTACGGACGTCTGAACGG
    ------------------X------------11---------X--------12----


    R endendend SalI
    ----------->
    GTTAATGATAG                  FIG.2
    CAATTACTATCAGCTG                                      196
    --------------->
```

FIG.3

FIG.4

FIG. 5

```
78    80                                    90   92   94        97
 T  V  A  A  C  N  L  P  I  V  R  G  P  C  R  A  F  I  Q  L
                                      <------A---------------
ACTGTGGCGGCCTGCAATCTCCCCATAGTCCGGGGCCCCTGCCGAGCCTTCATCCAGCTC
TGACACCGCCGGACGTTAGAGGGGTATCAGGCCCCGGGGACGGACCGGAAGTAGGTCGAG
                                    <-----------B---------------

     100                            110
 W  A  F  D  A  V  K  G  K  C  V  L  F  P  Y  G  G  C  Q  G
---------------------->< ----------C-------------------------
TGGGCATTTGATGCTGTCAAGGGGAAGTGCGTCCTCTTCCCCTACGGGGGGCTGCCAGGGC
ACCCGTAAACTACGACAGTTCCCCTTCACGCAGGAGAAGGGGATGCCCCCGACGGTCCCG
--------------->< -------------------D-------------------------

     120                            130
 N  G  N  K  F  Y  S  E  K  E  C  R  E  Y  C  G  V  P  G  D
-------------->
AACGGGAACAAGTTCTACTCAGAGAAGGAGTGCAGAGAGTACTGCGGTGTCCCTGGTGAT
TTGCCCTTGTTCAAGATGAGTCTCTTCCTCACGTCTCTCATGACGCCACAGGGACCACTA
-------------->

     140                 147
 G  D  E  E  L  L  R  F  S  N
GGTGATGAGGAGCTGCTGCGCTTCTCCAACTGACAACTGGCCGGTCTGCAAGTCAGAGGA
CCACTACTCCTCGACGACGCGAAGAGGTTGACTGTTGACCGGCCAGACGTTCAGTCTCCT

TGGCCAGTGTCTGTCCCGGGGTCCTGTGGCAGGCAGCGCCAAGCAACCTGGGTCCAAATA
ACCGGTCACAGACAGGGCCCCAGGACACCGTCCGTCGCGGTTCGTTGGACCCAGGTTTAT

AAAACTAAATTGTAAACTCCTGAAAAAAAAAAAAAAAAAA
TTTTGATTTAACATTTGAGGACTTTTTTTTTTTTTTTTTT
```

FIG.6

21

FIG.7

FIG.8

FIG. 9

FIG.10

PstI

EcoRI
PstI

pCY 17

SalI

PstI

HindIII

PstI
ACT GCA GTT - - - - - - - GGG GTA | TCT | TTG GAT AAA AGA GAG GCT GAA GCT

gly val | ser | leu asp lys arg glu ala glu ala

HindIII

site-directed
mutagenesis

PstI
ACT GCA GTT - - - - - - - GGG GTA | AGC | TTG - - - - - - - - - GAA GCT

gly val | ser | leu

glu ala

HindIII

|———— 213 bp ————| HindIII

PstI
ACT GCA GTT τ - - - - - - - GGG GTA

gly val

FIG.11

(I)

FIG.12

FIG. 13

FIG.14

FIG.15

EcoRI

pre-pro-α-factor

| lys | arg |
|-----|-----|
| AAA | AGA |

  1   2   36  38  92  94
ala val leu leu leu leu
GCT GTG···CTA-CTA···CTA-CTA···bikunin

EcoRI

PstI

PstI

PstI

p4LB

PstI

HindIII

Xhol

HindIII

BamHI

terminator

2 μ

PstI

URA3

FIG.16

EP 0 401 508 B1

FIG.17